# EUROPEAN PATENT APPLICATION

(11) **EP 4 282 429 A1**
(43) Date of publication of application: **29.11.2023**
(21) Application number: 22175424.5
(22) Date of filing: 25.05.2022
(51) Int. Cl.: A61K 39/12, C07K 14/005, C12N 15/86

(54) **MULTIVALENT COVID-19 VACCINES BASED ON ADENOVIRAL VECTORS**

(71) Applicant: Serum Institute of India Pvt. Ltd., Pune 411 028 (IN)
(72) Inventor: Shaligram, Umesh Sharadrao, 411009 Pune (IN); Narwade, Santosh Chandrakant, 411045 Pune (IN)
(74) Representative: Weickmann & Weickmann PartmbB

(57) **Abstract**

The present invention relates to the construction of Adenovirus-based vectors und to their use for inducing a broad, robust and durable cellular and humoral immune response in a subject.

## Description

The present invention relates to the construction of Adenovirus-based vectors und to their use for inducing a broad, robust and durable cellular and humoral immune response in a subject.

Corona Virus Disease 2019 (COVID-19) is a serious infectious disease caused by a member of the corona virus family, the Severe Acute Respiratory Syndrome Coronavirus 2 (SARS-CoV-2). Since its outbreak in Wuhan China in 2019, the disease has spread worldwide, leading to an ongoing pandemic. Numerous attempts to treat the disease have so far not had sufficient success. Despite medical interventions, the mortality rate is significant, particularly in elderly, immune compromised individuals. While work is underway to develop drugs that inhibit the virus, the primary treatment is symptomatic. Management involves the treatment of symptoms, supportive care, isolation, and experimental measures. Several COVID-19 vaccines have been approved and distributed in various countries. Among them, there are vaccines are based on mRNA and adenoviral platforms to express the spike protein of the ancestral SARS-CoV-2 and elicit neutralizing antibody responses against the Wuhan strain.

However, since the original appearance in Wuhan, several virus variants of concern have emerged that have multiple mutations, especially in the spike protein. Of particular importance are mutations within the receptor-binding domain (RBD) that reduce their neutralization by antibodies present in convalescent or vaccine-induced sera. Several first-generation vaccines have demonstrated reduced effectiveness in protecting from COVID-19 caused by these emerging variants of concern. So far, no vaccines adapted to SARS-CoV-2 variants have been authorized. An alternative strategy is to develop recombinant viral vectored multivalent vaccines. Besides the spike protein, such vaccines may express additional conserved SARS-CoV-2 antigens to broaden T cell immunity. Since antigenic changes in conserved, internal viral proteins that are the primary focus of T cell responses are improbable in SARS-CoV-2 viruses including variants of concern, such multivalent vaccines do not require frequent updating while they can boost the spike-specific immunity induced by first-generation vaccines. Thus, these vaccines are expected to be effective against both ancestral and variants of SARS-CoV-2.

However, there is still a need to provide improved vaccine compositions and methods that render a therapeutic effect, reduce or prevent viral entry into a cell, reduce direct and indirect toxicity of the virus to the patient, and produce an immune response that is effective to clear the virus from the patient. Advantageously, the vaccines should be able to provide protection against emerging new virus variants.

The present invention provides a solution to this problem by disclosing a replication defective adenoviral vector comprising
(i) a nucleic acid encoding at least one SARS-CoV-2 spike protein or a derivative thereof and
(ii) a nucleic acid encoding at least one SARS-CoV-2 nucleocapsid protein or a derivative thereof,
wherein the adenoviral vector displays a polypeptide comprising a receptor-binding domain (RBD) of a SARS-CoV-2 spike protein or a derivative thereof via protein IX (pIX) on its capsid.

SARS-CoV-2 is a virus of the species severe acute respiratory syndrome-related coronavirus (SARS-CoV). It is a positive-sense single-stranded RNA (+ssRNA) virus, with a single linear RNA segment. Like other coronaviruses, SARS-CoV-2 has four structural proteins, known as the S (spike), E (envelope), M (membrane), and N (nucleocapsid) proteins; the N protein holds the RNA genome, and the S, E, and M proteins together create the viral envelope. Coronavirus S proteins are glycoproteins, and type I membrane proteins (membranes containing a single transmembrane domain oriented on the extracellular side). They are divided into two functional parts (S1 and S2). The spike protein is the protein responsible for allowing the virus to attach to and fuse with the membrane of a host cell. The N protein forms complexes with genomic RNA, interacts with the viral membrane protein during virion assembly and plays a critical role in enhancing the efficiency of virus transcription and assembly.

Adenoviruses are among the most commonly used vectors for the delivery of genetic material into human cells. They have been used as vaccine delivery vehicles and to express recombinant proteins in cell lines and tissues. Adenoviral vectors characteristically induce strong cellular responses against their encoded transgenes, compared to recombinant, adjuvanted proteins that induce strong antibody responses. Those antibody responses alone may, however not suffice to provide protection against complex pathogens where a synergistic T cell response has proven to be beneficial.

The adenoviral vector of the present invention encodes spike proteins because of the potential to induce antibodies to against the RBD to neutralize the ability of the virus to bind host cells. More specifically, the SARS-CoV-2 spike protein is presented in two ways, firstly in the form of a nucleic acid encoding at least one SARS-CoV-2 spike protein or a derivative thereof, and secondly in the form of a polypeptide comprising a receptor-binding domain (RBD) of a SARS-CoV-2 spike protein or a derivative thereof displayed on the adenovirus capsid via protein IX (pIX). Due to this special characteristic, the adenoviral vector of the present invention combines the benefits of genetic and protein-based vaccination within one vaccine construct. Transgene-expression and producibility of the vector are not impeded by the pIX-display. The minor capsid protein IX (pIX) tolerates fusion of relatively large, functional proteins to its surface-exposed C-terminus without drastically decreasing its function. Compared to immunization with soluble protein, fusion of the RBD of the SARS-CoV-2 spike protein or a derivative thereof to pIX may ensure presentation of the antigen in its native conformation and in a highly repetitive nature inherent to viral capsids, both of which were shown to deliver strong activation signals to the recognizing B-cells.

The RBD of the SARS-CoV-2 spike protein or a derivative thereof can be attached with or without a linker. For example, a flexible glycine linker or a spacer can be used. Accessibility of the RBD of the SARS-Cov-2 spike protein or a derivative thereof on the surface of the adenoviral vector particle may be improved by the pIX- fusion via a linker and/or a spacer in comparison to direct fusion. The linker may be acting as a molecular hinge, making the antigen more flexible in its interaction with the antigen-binding domains of the B-cell receptor. A spacer molecule may ensure display of the antigen over its full length at an optimal distance from the surface of the adenovirus capsid by lifting the antigen up towards the surface.

Another characteristic feature of the adenoviral vector of the present invention is that in addition to the spike protein, an additional antigen is provided against which responses can be elicited. The adenoviral vector comprises a nucleic acid encoding at least one SARS-CoV-2 nucleocapsid protein or a derivative thereof. Thereby, the risk of the emergence of new strains of the virus with mutations in spike is addressed. The nucleocapsid protein is a highly conserved but is known to have variant regions in the emerging virus variants of concern. The terms "nucleocapsid protein," "nucleoprotein," and "nucleocapsid" are used interchangeably throughout this disclosure. Nucleocapsid associates with viral RNA within the virus and has a role in viral RNA replication, virus particle assembly, and release. The SARS-CoV-2 nucleocapsid was found to be a highly antigenic protein and patients infected with SARS-CoV-2 show antibody responses to the nucleocapsid. In addition, the nucleocapsid was found to particularly induce T-cell immunity. The T-cell based responses are critical given the finding that neutralizing antibody titers decline in COVID-19 patients after about 3 months. T cells are an important part of long-term immunity.

The at least one SARS-CoV-2 spike protein of (i) can be selected from spike proteins of any known SARS-CoV-2 viruses derived from the Wuhan variant and derivatives thereof. The spike protein of the original Wuhan coronavirus is preferably not included. In other words, the at least one spike protein of (i) relates preferably to any SARS-CoV-2 spike protein excluding the Wuhan coronavirus spike protein.

As used herein and in accordance with NCBI taxonomy database, the terms "original Wuhan coronavirus", "Wuhan variant", "2019-nCoV", "COVID-19", "COVID-19 virus", "Wuhan seafood market pneumonia virus" can be used interchangeably herein.

Five SARS-CoV-2 variants have been designated as variants of concern by the World Health Organization: the Alpha, Beta, Gamma, Delta, and Omicron variants. Thus, according to a preferred embodiment, the at least one SARS-CoV-2 spike protein of (i) comprises the spike protein of at least one of these variants of concern or derivatives thereof. Also included are the spike proteins of any subtypes of the above variants of concern, such as the Delta Plus variant and Omicron subvariants B.1.1.529 and BA.2.

According to a particularly preferred embodiment, the at least one SARS-CoV-2 spike protein of (i) comprises the spike protein of SARS-CoV-2 Omicron variant or a derivative thereof, SARS-CoV-2 Delta Plus variant or a derivative thereof, or any combinations thereof.

However, it is understood that the at least one SARS-CoV-2 spike protein of (i) can also be selected from spike proteins of other emerging SARS-CoV-2 variants, such as lineages B.1.1.207, B.1.1.317, B.1.616, B.1.618, B.1.640.2, AZ.5, C.1.2, B.1.429, B.1.427, C.37 (Lambda variant), B.1.621 (Mu variant), CAL.20C (Epsilon variant), B.1.617.1 (Kappa variant), B.1.526 (Iota variant), P.2 (Zeta variant), P.3 (Theta variant), B.1.525 (Eta variant), B.1.630, and B.1.640, as well as derivatives thereof and combinations of spike proteins from two or more different variants.

The respective sequences of the spike proteins can be taken from common data bases.

The term "derivatives" of the respective SARS-CoV-2 spike proteins is understood herein to mean proteins that deviate from the respective spike proteins in their sequence without losing the desired functionality. In addition, deviations in the sequence, for example in the form of substitutions, deletions or additions, are possible. For example, a derivative may have a shortened sequence compared to the complete spike protein, while preferably retaining the RBD. For example, the identity of the sequence of a derivative to the sequence of the complete spike protein may be at least 50%, at least 75%, at least 85%, at least 90%, at least 95% or at least 99%, over the entire length of the sequence.

The at least one nucleocapsid protein of (ii) can be selected from nucleocapsid proteins of any known SARS-CoV-2 viruses derived from the Wuhan variant and derivatives thereof, in particular those described above for the spike protein of (i). For example, it can be a nucleocapsid protein from a SARS-CoV-2 virus variant of concern such as the Alpha, Beta, Gamma, Delta, and Omicron variants or any subtypes thereof, e.g. the Delta Plus variant and Omicron subvariants B.1.1.529 and BA.2. The nucleocapsid protein of the original Wuhan virus is preferably not included. In other words, the at least one nucleocapsid protein of (ii) relates preferably to any SARS-CoV-2 nucleocapsid protein excluding the Wuhan coronavirus nucleocapsid protein.

According to a particularly preferred embodiment, the at least one SARS-CoV-2 nucleocapsid protein of (ii) comprises the nucleocapsid protein of SARS-CoV-2 Omicron variant or a derivative thereof, SARS-CoV-2 Delta Plus variant or a derivative thereof, or combinations thereof.

The respective sequences of the nucleocapsid proteins can be taken from common data bases.

The term "derivatives" of the respective SARS-CoV-2 nucleocapsid proteins is understood herein to mean proteins that deviate from the respective nucleocapsid proteins in their sequence without losing the desired functionality. In addition, deviations in the sequence, for example in the form of substitutions, deletions or additions, are possible. For example, a derivative may have a shortened sequence compared to the complete nucleocapsid protein. For example, the identity of the sequence of a derivative to the sequence of the complete nucleocapsid protein may be at least 50%, at least 75%, at least 85%, at least 90%, at least 95% or at least 99%, over the entire length of the sequence.

The RBD displayed on the capsid can be selected from RBDs of any known spike proteins of SARS-CoV-2 viruses derived from the Wuhan variant and derivatives thereof, in particular those described above for the spike protein of (i). For example, it can be a RBD of the spike protein from a SARS-CoV-2 virus variant of concern such as the Alpha, Beta, Gamma, Delta, and Omicron variants or any subtypes thereof, e.g. the Delta Plus variant and Omicron subvariants B.1.1.529 and BA.2. The respective sequences of the RBD can be taken from common data bases. The RBD of the spike protein of the original Wuhan virus is preferably not included. In other words, the RBD displayed on the capsid can be selected from RBDs of any known spike proteins of SARS-CoV-2 viruses excluding the Wuhan coronavirus spike protein.

According to a particularly preferred embodiment, the RBD displayed on the capsid is the RBD of SARS-CoV-2 Delta Plus variant or a derivative thereof.

Adenoviruses have been isolated from a large number of different species, and more than 100 different human serotypes have been reported. The overall organization of the Adenoviral genome is conserved among serotypes, such that specific functions are similarly positioned. Most adults have been exposed to the Adenovirus serotypes most commonly used in therapy. While vectors based on human Adenovirus 5 (HAdV5) are most frequently used, their immunogenicity is impaired by high levels of pre-existing immunity. Alternative Adenovirus serotypes such as HAdV35, HAdV26 and ChAdV3 are highly immunogenic while less sero-prevalent and unaffected by pre-existing immunity to HAdV5, making them suitable as vaccine vectors. The adenoviral vector of the present invention can be derived from adenoviruses of different species, preferably human or chimpanzee. The serotype of the adenovirus can for example be 5, 26, 35 or 3, in particular human Adenovirus serotype 5, 26 or 35 and chimpanzee Adenovirus serotype 3.

The adenoviral vector of the present invention is replication deficient. According to a preferred embodiment, it has deletions at least in the E1, and/or E3 gene regions and optionally one or more additional regions such as the E2b region. The sequences of wild type Adenovirus genome and the respective E1 and E3 regions are described in common databases. For example, the wild type Adenovirus 5 genome is described in Gen Bank Accession No. AY339865.1.

The term "deleted", or "deletion", as used herein in context with deletions in the E1, or E3 gene regions, refers to a specific DNA sequence that is mutated in such a way so as to prevent expression and/or function of at least one E1, or E3 gene product. Thus, in certain embodiments, "deleted" is used in relation to a specific DNA sequence that is deleted (removed) from the Ad genome. E1/E3 deleted or "containing a deletion within the E1/E3 region" refers to a deletion of at least one base pair within the respective region of the Ad genome. Thus, in certain embodiments, more than one base pair is deleted and in further embodiments, at least 20, 30, 40, 50, 60, 70, 80, 90, 100, 110, 120, 130, 140, or 150 base pairs are deleted. In another embodiment, the deletion is of more than 150, 160, 170, 180, 190, 200, 250, or 300 base pairs within the E1, and/or E3 region of the Ad genome, respectively. A deletion may be a deletion that prevents expression and/or function of at least one gene product and therefore, encompasses deletions within exons of encoding portions of E1E3-specific proteins as well as deletions within promoter and leader sequences. In a further embodiment, "deleted" refers to one or more point mutations in the DNA sequence of this region of an Ad genome such that one or more encoded proteins is non-functional. Such mutations include residues that are replaced with a different residue leading to a change in the amino acid sequence that result in a nonfunctional protein.

Referring to human Adenovirus 5 serotype, a deletion in the E1 gene region may be a deletion of positions 454-3513 compared to the wild type Adenovirus 5 genome (GenBank: AY339865.1). A deletion in the E3 gene region may be a deletion of positions 28592-30465 compared to the wild type Adenovirus 5 genome (GenBank: AY339865.1).

The nucleic acid sequence of a particularly preferred adenoviral vector of the present invention is shown in SEQ ID NO:1.

The adenoviral vector of the present invention is optimized for immunogenicity. It is capable to provide robust, durable cell-mediated and humoral immunity against SARS-CoV-2 infection. Thus, in one embodiment, the present disclosure provides an adenoviral vector for use in the prevention or treatment of a COVID-19 disease.

According to a preferred embodiment, the inventive adenoviral vector comprises a further a nucleic acid encoding at least one protein or derivative thereof of a microorganism, which is different from SARS-CoV-2. Preferably, such microorgansim is responsible for an infectious disease. "Derivative" in the sense of the invention means any portion of a wild-type, mutated or engineered protein which can elicit an immune response resulting in immunogenicity. Microorganisms can be any bacteria, protozoa or viruses.

When provided prophylactically, the compositions of the present disclosure are administered in advance of the development of, or the detection of the development of, a coronavirus disease, with the goal of preventing, inhibiting or delaying the development of the coronavirus disease; and/or generally preventing or inhibiting progression of the coronavirus disease in an individual. Therefore, prophylactic compositions can be administered to individuals that appear to be coronavirus disease free (healthy, or normal, individuals), or to individuals who have not yet been detected of coronavirus. Individuals who are at high risk for developing a coronavirus disease, may be treated prophylactically with a composition of the present disclosure.

When provided therapeutically, the compositions of the present invention are administered to an individual who is diagnosed with a coronavirus disease, with the goal of ameliorating or curing the coronavirus disease; increasing survival of the individual; preventing, inhibiting, reversing or delaying development of coronavirus disease in the individual.

Routes and frequency of administration of the therapeutic compositions described herein, as well as dosage, may vary from individual to individual, and may be readily established using standard techniques.

The administration of the adenoviral vector can be through a variety of suitable paths for delivery. One preferred route contemplated herein is by injection, such as intramuscular injection, intravenous injection or subcutaneous injection. In some embodiments, intramuscular or subcutaneous injection is preferred. Intramuscular administration is particularly favorable to optimize immunogenicity with the direct delivery of the adenoviral vector to the blood supply in the muscle to induce systemic immunity.

Another possible route is by administration to the respiratory tract. For protective immunity against mucosal pathogens, (i.e., SARS coronaviruses) immune activation in mucosal tissues instead of the more common approach of tolerance to maintain mucosal homeostasis allows for enhanced mucosal immune responses and better local protection. Thus, in one aspect of the invention, the adenoviral vector may be administered to the respiratory tract of a subject, in particular to the mucosa of the respiratory tract to induce mucosal immunity in addition to cell-mediated and humoral immunity. This type of vaccination induces both mucosal immunity as well as systemic immunity.

Further, the adenoviral vector can be administered to a patient by more than one route of administration, e.g. to induce both local and systemic immune responses.

In a preferred aspect, administration of the adenoviral vector of the invention is capable to generate long-term T and B cell memory.

The invention further provides a vaccine composition comprising the adenoviral vector disclosed herein. The vaccine composition can be formulated in a pharmaceutically acceptable excipient suitable for administration to a subject. According to a preferred embodiment the vaccine may comprise further active agents, i.e. in particular further immunogenicity conferring agents. Such further immunogenicity conferring agents may be directed against the same target, the same virus, the same subtype or any other subtype or even any other virus.

The vaccine composition may be adapted for the preferred way of administration described above. Thus, in one embodiment, the present disclosure provides a vaccine composition adapted for injection, in particular for intramuscular or subcutaneous injection.

In another embodiment, the present disclosure provides a vaccine composition adapted for administration to the respiratory tract of a subject, in particular to the mucosa of the respiratory tract to induce mucosal immunity in addition to cell-mediated and humoral immunity. Accordingly, the vaccine composition can be adapted for oral, intranasal, or sublingual administration.

In a preferred aspect, the vaccine disclosed herein generates long-term T and B cell memory.

Also disclosed herein are methods for preventing and/or treating coronavirus diseases, and especially COVID-19. Preferably, the method includes using an adenoviral vector as defined above that encodes the nucleocapsid protein and the spike protein of the coronavirus and displays a polypeptide comprising a receptor-binding domain (RBD) of a SARS-CoV-2 spike protein or a derivative thereof via protein IX (pIX) on its capsid. The described method is able to induce a broader cellular immunity against SARS-CoV-2. The adenoviral vector can be administered to a subject individual in an immunogenic composition. The virus vaccine, thus administered, would induce the cellular expression of SARS-CoV-2 nucleocapsid and spike protein and a direct immune response against SARS-CoV-2 spike protein RBD on the virus surface. With that in place, the individual would have an immune response against it, and be vaccinated. Notably, as the spike protein and in particular the nucleocapsid protein are relatively conserved polypeptides, immune responses can be elicited for a variety of members of the coronavirus family.

The vaccine compositions described herein can be used as a universal booster vaccine to any anti-SARS-CoV-2 vaccine directed against the SARS-CoV-2 spike (S) and/or nucleocapsid (N) proteins. According to this aspect, the present invention provides a method of boosting an antigen-specific immune response in a mammal comprising administering to said mammal a sufficient amount of the adenoviral vector or the vaccine composition disclosed herein. This booster can work even in patients who were immunized with an SARS-CoV-2 spike or nucleoprotein encoding vaccine other than those described herein and broaden the immunogenicity of these patients.

In certain embodiments, the boost described herein is administered at least 7 days after the initial prime vaccination, for example at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 28 days, at least 35 days, or at least 42 days. Preferably, the boost is administered after at least 2 months, at least 3 months, at least 4 months, or later. Booster administration after about 3 month is in particular preferred. The boost as described herein can effectively improve both antibody production against SARS-CoV-2 and cell-mediated immunity against SARS-CoV-2. The efficacy of the booster vaccine can be measured by any standard quantification of immune response.

Further booster vaccine administration, such as a second or third booster administration, are possible.

In particular embodiments, the boost described herein is administered by injection, preferably by intramuscular or subcutaneous injection. In alternative embodiments, the boost described herein is administered to the respiratory tract of a subject, e.g. to the mucosa of the respiratory tract, in particular via intranasal or oral administration. According to an especially preferred embodiment, the boost is administered by injection, preferably by intramuscular or subcutaneous injection, after about three month.

According to a further aspect, the present invention relates to a platform, i.e. a widely applicable technology, for providing multivalent vaccines comprising at least one adenoviral vector according as herein described. According to a preferred embodiment, the inventive adenoviral vector comprises a further a nucleic acid encoding at least one protein or derivative thereof of a microorganism responsible for an infectious disease.

LOCUS       pAd5-CMV-Om_S-T2A-Om/DP_N-SV40        39814 bp
DEFINITION  pAd5-CMV-Om_S-T2A-Om/DP_N-SV40pA-pIX-DP_RBD, DNA 39814 bases.
FEATURES             Location/Qualifiers
     misc_feature    39807..39814
                     /label="PacI"
     misc_feature    38696..39556
                     /label="Ampicillin resistance gene"
     misc_feature    37909..38570
                     /label="pUC origin"
     misc_feature    37733..37740
                     /label="PacI"
     gene            7031..7699
                     /label="RBD (Delta plus)"
     gene            6611..7702
                     /label="pIX"
     misc_feature    6515..37732
                     /label="E3-deleted Ad5 genome, Ad5_3′ R-ITR"
     polyA_site      6252..6473
                     /label="SV40 late pA"
     misc_feature    6243..6251
                     /label="Triple stop codons"
     gene            4995..6242
                     /label="Nucleocapsid (Omicron/Delta plus hybrid)"
     misc_feature    4932..4994
                     /label="T2A"
     gene            1131..4931
                     /label="Spike (Omicron)"
     misc_feature    1125..1130
                     /label="Kozak"
     promoter        536..1124
                     /label="CMV Promoter"
     misc_feature    1..458
                     /label="Ad5_5′ L-ITR"
ORIGIN
        1  CATCATCAAT AATATACCTT ATTTTGGATT GAAGCCAATA TGATAATGAG
       51  GGGGTGGAGT TTGTGACGTG GCGCGGGGCG TGGGAACGGG GCGGGTGACG
      101  TAGTAGTGTG GCGGAAGTGT GATGTTGCAA GTGTGGCGGA ACACATGTAA
      151  GCGACGGATG TGGCAAAAGT GACGTTTTTG GTGTGCGCCG GTGTACACAG
      201  GAAGTGACAA TTTTCGCGCG GTTTTAGGCG GATGTTGTAG TAAATTTGGG
      251  CGTAACCGAG TAAGATTTGG CCATTTTCGC GGGAAAACTG AATAAGAGGA
      301  AGTGAAATCT GAATAATTTT GTGTTACTCA TAGCGCGTAA TATTTGTCTA
      351  GGGCCGCGGG GACTTTGACC GTTTACGTGG AGACTCGCCC AGGTGTTTTT
      401  CTCAGGTGTT TTCCGCGTTC CGGGTCAAAG TTGGCGTTTT ATTATTATAG
      451  TCAGTCGAAG CTTGGATCCG GTACCTCTAG AATTCTCGAG CGGCCGCTAG
      501  CGACATCGAT CCAAGTTTGT ACAAAAAAGC AGGCTTAGTT ATTAATAGTA
      551  ATCAATTACG GGGTCATTAG TTCATAGCCC ATATATGGAG TTCCGCGTTA
      601  CATAACTTAC GGTAAATGGC CCGCCTGGCT GACCGCCCAA CGACCCCCGC
      651  CCATTGACGT CAATAATGAC GTATGTTCCC ATAGTAACGC CAATAGGGAC
      701  TTTCCATTGA CGTCAATGGG TGGAGTATTT ACGGTAAACT GCCCACTTGG
      751  CAGTACATCA AGTGTATCAT ATGCCAAGTA CGCCCCCTAT TGACGTCAAT
      801  GACGGTAAAT GGCCCGCCTG GCATTATGCC CAGTACATGA CCTTATGGGA
      851  CTTTCCTACT TGGCAGTACA TCTACGTATT AGTCATCGCT ATTACCATGG
      901  TGATGCGGTT TTGGCAGTAC ATCAATGGGC GTGGATAGCG GTTTGACTCA
      951  CGGGGATTTC CAAGTCTCCA CCCCATTGAC GTCAATGGGA GTTTGTTTTG
     1001  GCACCAAAAT CAACGGGACT TTCCAAAATG TCGTAACAAC TCCGCCCCAT
     1051  TGACGCAAAT GGGCGGTAGG CGTGTACGGT GGGAGGTCTA TATAAGCAGA
     1101  GCTGGTTTAG TGAACCGTCA GATCGCCACC ATGTTCGTGT TCCTGGTGCT
     1151  GCTGCCTCTG GTGAGCTCTC AGTGCGTCAA CCTGACCACA CGGACCCAGC
     1201  TGCCCCCTGC CTACACAAAC AGCTTCACCC GGGGCGTGTA CTACCCCGAC
     1251  AAGGTGTTCC GGAGCTCCGT GCTGCACAGC ACCCAGGATC TGTTTCTGCC
     1301  CTTCTTCAGC AATGTGACCT GGTTCCACGT GATCTCCGGC ACAAACGGCA
     1351  CCAAGCGGTT CGACAACCCT GTGCTGCCCT TCAACGACGG CGTGTACTTT
     1401  GCTAGCATCG AGAAGAGCAA CATCATCCGC GGCTGGATCT TCGGCACCAC

1451 ACTGGACTCC AAGACCCAGA GCCTGCTGAT CGTGAATAAC GCTACCAACG
1501 TGGTGATCAA GGTGTGTGAA TTCCAGTTCT GCAACGACCC TTTCCTGGAT
1551 CACAAGAACA ATAAGAGCTG GATGGAGAGC GAGTTCCGGG TGTACTCCAG
1601 CGCCAACAAC TGCACCTTCG AATACGTGTC TCAGCCCTTC CTGATGGACC
1651 TGGAGGGCAA GCAGGGCAAC TTCAAGAACC TGCGGGAGTT TGTCTTCAAA
1701 AACATCGACG GCTACTTCAA GATCTACAGC AAGCACACCC CTATCATCGT
1751 GCGCGACCTG CCCCAGGGCT TCAGCGCCCT GGAACCTCTG GTGGACCTCC
1801 CCATCGGCAT TAATATCACC CGGTTCCAAA CACTGCTGGC CCTGCACCGG
1851 AGCTACCTGA CCCCAGGAGA TTCCTCTTCC GGATGGACCG CCGGCGCTGC
1901 CGCCTACTAT GTGGGCTACC TGCAGCCCCG GACCTTCCTG CTGAAGTACA
1951 ACGAGAACGG CACCATTACA GACGCCGTGG ACTGTGCCCT GGACCCTCTG
2001 AGCGAGACCA AGTGCACCCT GAAGAGCTTT ACAGTGGAGA AGGGCATCTA
2051 CCAGACCAGC AACTTCAGAG TGCAGCCCAC CGAGTCCATC GTGCGGTTCC
2101 CCAACATTAC CAATCTGTGC CCTTTCGACG AAGTCTTTAA CGCCACCCGG
2151 TTCGCCAGCG TGTACGCCTG GAACAGAAAA CGGATCAGCA ACTGTGTGGC
2201 CGATTATTCT GTGCTGTACA ACCTGGCCCC CTTCTTCACC TTCAAGTGCT
2251 ACGGCGTGAG CCCTACAAAG CTGAATGACC TGTGCTTTAC CAACGTGTAC
2301 GCCGACAGCT TCGTGATTAG AGGTGATGAA GTCCGGCAGA TTGCCCCCGG
2351 CCAGACCGGC AACATCGCCG ACTACAACTA CAAGCTGCCT GATGACTTCA
2401 CCGGCTGCGT GATCGCCTGG AACAGCAATA AGCTGGATTC CAAGGTGTCC
2451 GGCAACTACA ACTATCTGTA CCGGCTGTTC AGAAAGAGCA ATCTGAAGCC
2501 TTTCGAGCGG GACATCAGCA CCGAGATCTA CCAGGCCGGC AACAAGCCCT
2551 GTAATGGCGT GGCCGGATTT AACTGCTACT TCCCTCTGCG GTCTTATTCT
2601 TTTCGGCCTA CATACGGAGT GGGCCACCAG CCATACCGGG TGGTGGTGCT
2651 GTCTTTTGAA CTGCTGCATG CCCCTGCCAC AGTGTGCGGC CCTAAGAAGA
2701 GCACCAACCT GGTCAAGAAC AAGTGCGTGA ACTTCAATTT CAATGGCCTC
2751 AAGGGCACAG GCGTGCTGAC CGAATCTAAC AAGAAGTTCC TGCCTTTCCA
2801 GCAGTTCGGA CGGGACATCG CAGACACCAC AGATGCTGTG CGGGACCCCC
2851 AGACCCTGGA GATTCTGGAC ATCACACCTT GCAGCTTCGG CGGCGTGTCC
2901 GTGATCACCC CTGGCACAAA CACCAGCAAC CAGGTGGCCG TGCTGTACCA
2951 GGGCGTGAAC TGCACCGAGG TGCCCGTGGC CATCCACGCC GATCAGCTGA
3001 CCCCTACATG GAGAGTGTAC AGCACAGGCT CTAATGTGTT TCAAACACGG
3051 GCCGGCTGCC TGATCGGAGC TGAGTACGTC AATAACAGCT ACGAGTGCGA
3101 CATCCCCATT GGCGCCGGCA TCTGTGCCAG CTACCAGACA CAGACCAAGA
3151 GCCACCGGAG AGCCAGGAGC GTGGCCTCCC AGAGCATCAT TGCCTACACC
3201 ATGAGCCTGG GCGCCGAGAA CAGCGTGGCA TACAGCAACA ATTCCATCGC
3251 CATCCCTACA AACTTCACCA TCAGCGTGAC AACCGAAATC CTCCCCGTGA
3301 GCATGACCAA AACATCTGTG GACTGCACCA TGTACATCTG CGGCGATAGC
3351 ACCGAGTGTA GCAACCTGCT GCTGCAGTAC GGCTCCTTCT GCACCCAGCT
3401 GAAGCGGGCC CTGACAGGCA TCGCCGTGGA GCAGGACAAG AACACCCAGG
3451 AAGTGTTCGC CCAGGTGAAA CAGATCTACA AGACCCCCCC TATCAAGTAC
3501 TTCGGAGGCT TTAACTTCAG CCAGATCCTG CCTGACCCAA GCAAGCCCTC
3551 CAAACGGAGC TTCATCGAGG ATCTGCTGTT CAACAAGGTG ACCCTGGCCG
3601 ACGCCGGCTT TATCAAGCAG TACGGCGACT GCCTGGGCGA TATTGCCGCC
3651 CGGGACCTGA TCTGCGCCCA GAAGTTCAAG GGCCTGACAG TGCTGCCTCC
3701 CCTGCTGACC GACGAGATGA TCGCCCAGTA CACCAGCGCT CTGCTGGCCG
3751 GAACAATCAC ATCTGGATGG ACCTTCGGCG CTGGCGCAGC CCTGCAGATC
3801 CCTTTCGCCA TGCAGATGGC CTACCGGTTC AACGGCATCG GCGTGACCCA
3851 GAACGTGCTG TACGAAAACC AGAAGCTGAT CGCCAACCAG TTCAACAGCG
3901 CCATCGGCAA GATCCAGGAC AGCCTGTCCA GCACCGCCTC CGCCCTGGGA
3951 AAGCTGCAGG ACGTGGTGAA CCACAACGCC CAGGCCCTGA ATACACTGGT
4001 GAAACAGCTG TCTAGCAAAT TCGGGGCCAT CAGCAGCGTC CTGAACGATA
4051 TCTTTTCCCG GCTGGACCCC CCTGAGGCCG AGGTGCAGAT CGATAGGCTG
4101 ATCACCGGCC GGCTGCAGAG CCTGCAAACA TACGTGACCC AGCAGCTGAT
4151 CAGAGCCGCT GAGATCCGGG CCAGCGCTAA CCTGGCAGCC ACCAAGATGA
4201 GCGAGTGCGT GCTGGGCCAG TCTAAGCGGG TGGACTTCTG CGGCAAGGGA
4251 TACCACCTGA TGAGCTTCCC CCAGAGCGCC CCACACGGCG TGGTGTTCCT
4301 GCATGTGACC TACGTGCCCG CCCAGGAGAA GAACTTCACA ACCGCCCCTG
4351 CCATCTGTCA CGACGGCAAG GCCCACTTTC CTCGGGAGGG CGTGTTCGTC
4401 AGCAACGGGA CCCACTGGTT CGTGACACAG CGGAACTTCT ACGAGCCCCA
4451 GATCATCACA ACCGACAACA CCTTCGTGAG CGGCAACTGC GACGTGGTGA
4501 TCGGCATCGT CAACAACACC GTGTACGACC CTCTGCAACC CGAACTGGAT
4551 TCCTTCAAGG AGGAGCTGGA CAAGTACTTC AAAAATCACA CCAGCCCTGA

4601 CGTGGACCTG GGGGACATTA GCGGCATCAA CGCCTCCGTG GTGAACATCC
4651 AGAAGGAAAT CGATCGGCTG AATGAGGTGG CCAAGAATCT GAACGAGAGC
4701 CTGATCGACC TGCAGGAGCT GGGCAAGTAC GAGCAGTATA TCAAGTGGCC
4751 ATGGTACATC TGGCTGGGCT TTATCGCCGG ACTGATCGCT ATCGTGATGG
4801 TGACCATCAT GCTGTGCTGT ATGACCAGCT GCTGTAGCTG CCTGAAAGGC
4851 TGCTGCAGCT GTGGCTCCTG TTGCAAGTTC GACGAAGATG ACTCCGAGCC
4901 TGTGCTGAAA GGCGTGAAGC TGCACTACAC AGGAAGCGGA GAGGGCAGGG
4951 GAAGTCTTCT AACATGCGGG GACGTGGAGG AAAATCCCGG CCCCATGAGC
5001 GACAACGGCC CCCAGAATCA GAGAAACGCC CTGCGGATCA CCTTCGGCGG
5051 ACCTTCCGAC TCCACAGGCT CTAATCAAAA TGGAGGCGCT AGATCTAAGC
5101 AGCGGAGACC TCAGGGCCTG CCCAACAACA CCGCCAGCTG GTTTACAGCC
5151 CTGACCCAGC ACGGCAAGGA GGGACTGAAA TTCCCTCGGG GCCAGGGCGT
5201 GCCCATCAAT ACCAACTCCT CTCCTGATGA CCAGATTGGA TACTACAGAC
5251 GCGCTACAAG ACGGATCAGA GGCGGCGATG GCAAGATGAA GGACCTGAGC
5301 CCTCGGTGGT ACTTTTATTA CCTCGGAACA GGCCCCGAAG CCGGCCTGCC
5351 TTACGGCGCC AACAAGGATG GCATCATCTG GGTGGCAACC GAGGGAGCCC
5401 TGAATACACC CAAGGACCAC ATCGGCACAA GAAACCCTGC CAACAACGCC
5451 GCCATCGTCC TGCAGCTGCC ACAGGGCACC ACACTGCCTA AAGGATTTTA
5501 CGCTGAAGGT TCCCGGGGCG GCTCTCAGGC ATCTAGCAGA TCCAGCTCCC
5551 GGAGCAGAAA TTCCAGCAGA AACTCTACCC CTGGCAGCAG CAAGCGGACC
5601 TCCCCCGCTA GAATGGCCGG AAATGGCTGC GATGCCGCCC TGGCCCTGCT
5651 GCTGCTGGAT CGGCTGAACC AGCTGGAGTC CAAAATGAGC GGCAAGGGGC
5701 AGCAGCAGCA GGGGCAGACA GTGACCAAGA AATCCGCCGC CGAAGCCAGC
5751 AAGAAGCCTA GACAGAAGCG GACCGCCACC AAAGCCTACA ACGTGACACA
5801 AGCTTTTGGA AGGAGAGGCC CAGAACAGAC CCAGGGCAAC TTCGGCGACC
5851 AGGAGCTGAT CCGGCAGGGC ACCGATTACA AGCACTGGCC CCAGATCGCC
5901 CAGTTCGCCC CTTCTGCCAG CGCCTTCTTT GGAATGTCTA GAATCGGCAT
5951 GGAAGTGACA CCTAGCGGCA CCTGGCTGAC CTACACAGGC GCCATCAAGC
6001 TGGATGACAA GGACCCCAAC TTCAAGGATC AGGTGATCCT GCTGAATAAG
6051 CACATTGATG CCTACAAGAC CTTCCCCCCC ACAGAGCCCA AGAAGGACAA
6101 GAAAAAGAAG GCCTACGAGA CCCAGGCCCT GCCTCAGCGG CAGAAGAAAC
6151 AGCAGACCGT GACACTGCTG CCCGCCGCCG ATCTGGACGA TTTCTCCAAG
6201 CAGCTGCAGC AGAGCATGTC TAGCGCCGAC TCTACACAGG CCTGATAATA
6251 GCAGACATGA TAAGATACAT TGATGAGTTT GGACAAACCA CAACTAGAAT
6301 GCAGTGAAAA AAATGCTTTA TTTGTGAAAT TTGTGATGCT ATTGCTTTAT
6351 TTGTAACCAT TATAAGCTGC AATAAACAAG TTAACAACAA CAATTGCATT
6401 CATTTTATGT TTCAGGTTCA GGGGGAGGTG TGGGAGGTTT TTTAAAGCAA
6451 GTAAAACCTC TACAAATGTG GTAACCCAGC TTTCTTGTAC AAAGTGGGAT
6501 CGATTCGACA GATCACTGAA ATGTGTGGGC GTGGCTTAAG GGTGGGAAAG
6551 AATATATAAG GTGGGGGTCT TATGTAGTTT TGTATCTGTT TTGCAGCAGC
6601 CGCCGCCGCC ATGAGCACCA ACTCGTTTGA TGGAAGCATT GTGAGCTCAT
6651 ATTTGACAAC GCGCATGCCC CCATGGGCCG GGGTGCGTCA GAATGTGATG
6701 GGCTCCAGCA TTGATGGTCG CCCCGTCCTG CCCGCAAACT CTACTACCTT
6751 GACCTACGAG ACCGTGTCTG GAACGCCGTT GGAGACTGCA GCCTCCGCCG
6801 CCGCTTCAGC CGCTGCAGCC ACCGCCCGCG GGATTGTGAC TGACTTTGCT
6851 TTCCTGAGCC CGCTTGCAAG CAGTGCAGCT TCCCGTTCAT CCGCCCGCGA
6901 TGACAAGTTG ACGGCTCTTT TGGCACAATT GGATTCTTTG ACCCGGGAAC
6951 TTAATGTCGT TTCTCAGCAG CTGTTGGATC TGCGCCAGCA GGTTTCTGCC
7001 CTGAAGGCTT CCTCCCCTCC CAATGCGGTT CGGGTGCAGC CAACAGAAAG
7051 CATCGTGAGA TTCCCCAATA TCACCAACCT GTGTCCTTTC GGCGAGGTGT
7101 TCAACGCCAC CCGGTTCGCT AGCGTGTACG CCTGGAATAG AAAGCGGATC
7151 TCTAACTGCG TGGCCGATTA CAGCGTGCTG TACAACAGCG CTAGCTTCTC
7201 CACCTTCAAG TGCTACGGCG TGAGCCCAAC AAAGCTGAAC GACCTGTGTT
7251 TTACCAATGT GTACGCCGAT AGCTTCGTGA TTCGGGGAGA CGAGGTCAGA
7301 CAGATCGCCC CTGGCCAGAC CGGCAACATC GCAGACTACA ACTACAAGCT
7351 GCCCGATGAC TTCACAGGCT GCGTGATCGC CTGGAACAGC AACAATCTGG
7401 ATTCCAAGGT GGGCGGAAAC TACAATTACC GGTACAGACT GTTCAGAAAG
7451 AGCAACCTGA AGCCTTTCGA GCGGGACATC AGCACCGAGA TCTACCAGGC
7501 TGGCTCTAAA CCCTGCAACG GCGTGGAGGG ATTTAACTGC TACTTCCCTC
7551 TGCAGAGCTA CGGCTTCCAG CCCACCAATG GCGTGGGCTA CCAACCTTAC
7601 CGCGTGGTGG TGCTGAGCTT TGAACTGCTG CACGCCCCTG CCACCGTGTG
7651 TGGCCCCAAG AAGAGCACAA ACCTGGTGAA GAACAAGTGC GTGAACTTCT
7701 AAAACATAAA TAAAAAACCA GACTCTGTTT GGATTTGGAT CAAGCAAGTG

7751 TCTTGCTGTC TTTATTTAGG GGTTTTGCGC GCGCGGTAGG CCCGGGACCA
7801 GCGGTCTCGG TCGTTGAGGG TCCTGTGTAT TTTTTCCAGG ACGTGGTAAA
7851 GGTGACTCTG GATGTTCAGA TACATGGGCA TAAGCCCGTC TCTGGGGTGG
7901 AGGTAGCACC ACTGCAGAGC TTCATGCTGC GGGGTGGTGT TGTAGATGAT
7951 CCAGTCGTAG CAGGAGCGCT GGGCGTGGTG CCTAAAAATG TCTTTCAGTA
8001 GCAAGCTGAT TGCCAGGGGC AGGCCCTTGG TGTAAGTGTT TACAAAGCGG
8051 TTAAGCTGGG ATGGGTGCAT ACGTGGGGAT ATGAGATGCA TCTTGGACTG
8101 TATTTTTAGG TTGGCTATGT TCCCAGCCAT ATCCCTCCGG GGATTCATGT
8151 TGTGCAGAAC CACCAGCACA GTGTATCCGG TGCACTTGGG AAATTTGTCA
8201 TGTAGCTTAG AAGGAAATGC GTGGAAGAAC TTGGAGACGC CCTTGTGACC
8251 TCCAAGATTT TCCATGCATT CGTCCATAAT GATGGCAATG GGCCCACGGG
8301 CGGCGGCCTG GGCGAAGATA TTTCTGGGAT CACTAACGTC ATAGTTGTGT
8351 TCCAGGATGA GATCGTCATA GGCCATTTTT ACAAAGCGCG GGCGGAGGGT
8401 GCCAGACTGC GGTATAATGG TTCCATCCGG CCCAGGGGCG TAGTTACCCT
8451 CACAGATTTG CATTTCCCAC GCTTTGAGTT CAGATGGGGG GATCATGTCT
8501 ACCTGCGGGG CGATGAAGAA AACGGTTTCC GGGGTAGGGG AGATCAGCTG
8551 GGAAGAAAGC AGGTTCCTGA GCAGCTGCGA CTTACCGCAG CCGGTGGGCC
8601 CGTAAATCAC ACCTATTACC GGGTGCAACT GGTAGTTAAG AGAGCTGCAG
8651 CTGCCGTCAT CCCTGAGCAG GGGGGCCACT TCGTTAAGCA TGTCCCTGAC
8701 TCGCATGTTT TCCCTGACCA AATCCGCCAG AAGGCGCTCG CCGCCCAGCG
8751 ATAGCAGTTC TTGCAAGGAA GCAAAGTTTT TCAACGGTTT GAGACCGTCC
8801 GCCGTAGGCA TGCTTTTGAG CGTTTGACCA AGCAGTTCCA GGCGGTCCCA
8851 CAGCTCGGTC ACCTGCTCTA CGGCATCTCG ATCCAGCATA TCTCCTCGTT
8901 TCGCGGGTTG GGGCGGCTTT CGCTGTACGG CAGTAGTCGG TGCTCGTCCA
8951 GACGGGCCAG GGTCATGTCT TTCCACGGGC GCAGGGTCCT CGTCAGCGTA
9001 GTCTGGGTCA CGGTGAAGGG GTGCGCTCCG GGCTGCGCGC TGGCCAGGGT
9051 GCGCTTGAGG CTGGTCCTGC TGGTGCTGAA GCGCTGCCGG TCTTCGCCCT
9101 GCGCGTCGGC CAGGTAGCAT TTGACCATGG TGTCATAGTC CAGCCCCTCC
9151 GCGGCGTGGC CCTTGGCGCG CAGCTTGCCC TTGGAGGAGG CGCCGCACGA
9201 GGGGCAGTGC AGACTTTTGA GGGCGTAGAG CTTGGGCGCG AGAAATACCG
9251 ATTCCGGGGA GTAGGCATCC GCGCCGCAGG CCCCGCAGAC GGTCTCGCAT
9301 TCCACGAGCC AGGTGAGCTC TGGCCGTTCG GGGTCAAAAA CCAGGTTTCC
9351 CCCATGCTTT TTGATGCGTT TCTTACCTCT GGTTTCCATG AGCCGGTGTC
9401 CACGCTCGGT GACGAAAAGG CTGTCCGTGT CCCCGTATAC AGACTTGAGA
9451 GGCCTGTCCT CGAGCGGTGT TCCGCGGTCC TCCTCGTATA GAAACTCGGA
9501 CCACTCTGAG ACAAAGGCTC GCGTCCAGGC CAGCACGAAG GAGGCTAAGT
9551 GGGAGGGGTA GCGGTCGTTG TCCACTAGGG GGTCCACTCG CTCCAGGGTG
9601 TGAAGACACA TGTCGCCCTC TTCGGCATCA AGGAAGGTGA TTGGTTTGTA
9651 GGTGTAGGCC ACGTGACCGG GTGTTCCTGA AGGGGGGCTA TAAAAGGGGG
9701 TGGGGGCGCG TTCGTCCTCA CTCTCTTCCG CATCGCTGTC TGCGAGGGCC
9751 AGCTGTTGGG GTGAGTACTC CCTCTGAAAA GCGGGCATGA CTTCTGCGCT
9801 AAGATTGTCA GTTTCCAAAA ACGAGGAGGA TTTGATATTC ACCTGGCCCG
9851 CGGTGATGCC TTTGAGGGTG GCCGCATCCA TCTGGTCAGA AAAGACAATC
9901 TTTTTGTTGT CAAGCTTGGT GGCAAACGAC CCGTAGAGGG CGTTGGACAG
9951 CAACTTGGCG ATGGAGCGCA GGGTTTGGTT TTTGTCGCGA TCGGCGCGCT
10001 CCTTGGCCGC GATGTTTAGC TGCACGTATT CGCGCGCAAC GCACCGCCAT
10051 TCGGGAAAGA CGGTGGTGCG CTCGTCGGGC ACCAGGTGCA CGCGCCAACC
10101 GCGGTTGTGC AGGGTGACAA GGTCAACGCT GGTGGCTACC TCTCCGCGTA
10151 GGCGCTCGTT GGTCCAGCAG AGGCGGCCGC CCTTGCGCGA GCAGAATGGC
10201 GGTAGGGGGT CTAGCTGCGT CTCGTCCGGG GGGTCTGCGT CCACGGTAAA
10251 GACCCCGGGC AGCAGGCGCG CGTCGAAGTA GTCTATCTTG CATCCTTGCA
10301 AGTCTAGCGC CTGCTGCCAT GCGCGGGCGG CAAGCGCGCG CTCGTATGGG
10351 TTGAGTGGGG GACCCCATGG CATGGGGTGG GTGAGCGCGG AGGCGTACAT
10401 GCCGCAAATG TCGTAAACGT AGAGGGGCTC TCTGAGTATT CCAAGATATG
10451 TAGGGTAGCA TCTTCCACCG CGGATGCTGG CGCGCACGTA ATCGTATAGT
10501 TCGTGCGAGG GAGCGAGGAG GTCGGGACCG AGGTTGCTAC GGGCGGGCTG
10551 CTCTGCTCGG AAGACTATCT GCCTGAAGAT GGCATGTGAG TTGGATGATA
10601 TGGTTGGACG CTGGAAGACG TTGAAGCTGG CGTCTGTGAG ACCTACCGCG
10651 TCACGCACGA AGGAGGCGTA GGAGTCGCGC AGCTTGTTGA CCAGCTCGGC
10701 GGTGACCTGC ACGTCTAGGG CGCAGTAGTC CAGGGTTTCC TTGATGATGT
10751 CATACTTATC CTGTCCCTTT TTTTTCCACA GCTCGCGGTT GAGGACAAAC
10801 TCTTCGCGGT CTTTCCAGTA CTCTTGGATC GGAAACCCGT CGGCCTCCGA
10851 ACGGTAAGAG CCTAGCATGT AGAACTGGTT GACGGCCTGG TAGGCGCAGC

10901 ATCCCTTTTC TACGGGTAGC GCGTATGCCT GCGCGGCCTT CCGGAGCGAG
10951 GTGTGGGTGA GCGCAAAGGT GTCCCTGACC ATGACTTTGA GGTACTGGTA
11001 TTTGAAGTCA GTGTCGTCGC ATCCGCCCTG CTCCCAGAGC AAAAAGTCCG
11051 TGCGCTTTTT GGAACGCGGA TTTGGCAGGG CGAAGGTGAC ATCGTTGAAG
11101 AGTATCTTTC CCGCGCGAGG CATAAAGTTG CGTGTGATGC GGAAGGGTCC
11151 CGGCACCTCG GAACGGTTGT TAATTACCTG GGCGGCGAGC ACGATCTCGT
11201 CAAAGCCGTT GATGTTGTGG CCCACAATGT AAAGTTCCAA GAAGCGCGGG
11251 ATGCCCTTGA TGGAAGGCAA TTTTTTAAGT TCCTCGTAGG TGAGCTCTTC
11301 AGGGGAGCTG AGCCCGTGCT CTGAAAGGGC CCAGTCTGCA AGATGAGGGT
11351 TGGAAGCGAC GAATGAGCTC CACAGGTCAC GGGCCATTAG CATTTGCAGG
11401 TGGTCGCGAA AGGTCCTAAA CTGGCGACCT ATGGCCATTT TTTCTGGGGT
11451 GATGCAGTAG AAGGTAAGCG GGTCTTGTTC CCAGCGGTCC CATCCAAGGT
11501 TCGCGGCTAG GTCTCGCGCG GCAGTCACTA GAGGCTCATC TCCGCCGAAC
11551 TTCATGACCA GCATGAAGGG CACGAGCTGC TTCCCAAAGG CCCCCATCCA
11601 AGTATAGGTC TCTACATCGT AGGTGACAAA GAGACGCTCG GTGCGAGGAT
11651 GCGAGCCGAT CGGGAAGAAC TGGATCTCCC GCCACCAATT GGAGGAGTGG
11701 CTATTGATGT GGTGAAAGTA GAAGTCCCTG CGACGGGCCG AACACTCGTG
11751 CTGGCTTTTG TAAAAACGTG CGCAGTACTG GCAGCGGTGC ACGGGCTGTA
11801 CATCCTGCAC GAGGTTGACC TGACGACCGC GCACAAGGAA GCAGAGTGGG
11851 AATTTGAGCC CCTCGCCTGG CGGGTTTGGC TGGTGGTCTT CTACTTCGGC
11901 TGCTTGTCCT TGACCGTCTG GCTGCTCGAG GGGAGTTACG GTGGATCGGA
11951 CCACCACGCC GCGCGAGCCC AAAGTCCAGA TGTCCGCGCG CGGCGGTCGG
12001 AGCTTGATGA CAACATCGCG CAGATGGGAG CTGTCCATGG TCTGGAGCTC
12051 CCGCGGCGTC AGGTCAGGCG GGAGCTCCTG CAGGTTTACC TCGCATAGAC
12101 GGGTCAGGGC GCGGGCTAGA TCCAGGTGAT ACCTAATTTC CAGGGGCTGG
12151 TTGGTGGCGG CGTCGATGGC TTGCAAGAGG CCGCATCCCC GCGGCGCGAC
12201 TACGGTACCG CGCGGCGGGC GGTGGGCCGC GGGGGTGTCC TTGGATGATG
12251 CATCTAAAAG CGGTGACGCG GGCGAGCCCC CGGAGGTAGG GGGGGCTCCG
12301 GACCCGCCGG GAGAGGGGGC AGGGGCACGT CGGCGCCGCG CGCGGGCAGG
12351 AGCTGGTGCT GCGCGCGTAG GTTGCTGGCG AACGCGACGA CGCGGCGGTT
12401 GATCTCCTGA ATCTGGCGCC TCTGCGTGAA GACGACGGGC CCGGTGAGCT
12451 TGAGCCTGAA AGAGAGTTCG ACAGAATCAA TTTCGGTGTC GTTGACGGCG
12501 GCCTGGCGCA AAATCTCCTG CACGTCTCCT GAGTTGTCTT GATAGGCGAT
12551 CTCGGCCATG AACTGCTCGA TCTCTTCCTC CTGGAGATCT CCGCGTCCGG
12601 CTCGCTCCAC GGTGGCGGCG AGGTCGTTGG AAATGCGGGC CATGAGCTGC
12651 GAGAAGGCGT TGAGGCCTCC CTCGTTCCAG ACGCGGCTGT AGACCACGCC
12701 CCCTTCGGCA TCGCGGGCGC GCATGACCAC CTGCGCGAGA TTGAGCTCCA
12751 CGTGCCGGGC GAAGACGGCG TAGTTTCGCA GGCGCTGAAA GAGGTAGTTG
12801 AGGGTGGTGG CGGTGTGTTC TGCCACGAAG AAGTACATAA CCCAGCGTCG
12851 CAACGTGGAT TCGTTGATAT CCCCCAAGGC CTCAAGGCGC TCCATGGCCT
12901 CGTAGAAGTC CACGGCGAAG TTGAAAAACT GGGAGTTGCG CGCCGACACG
12951 GTTAACTCCT CCTCCAGAAG ACGGATGAGC TCGGCGACAG TGTCGCGCAC
13001 CTCGCGCTCA AAGGCTACAG GGGCCTCTTC TTCTTCTTCA ATCTCCTCTT
13051 CCATAAGGGC CTCCCCTTCT TCTTCTTCTG GCGGCGGTGG GGGAGGGGGG
13101 ACACGGCGGC GACGACGGCG CACCGGGAGG CGGTCGACAA AGCGCTCGAT
13151 CATCTCCCCG CGGCGACGGC GCATGGTCTC GGTGACGGCG CGGCCGTTCT
13201 CGCGGGGGCG CAGTTGGAAG ACGCCGCCCG TCATGTCCCG GTTATGGGTT
13251 GGCGGGGGGC TGCCATGCGG CAGGGATACG GCGCTAACGA TGCATCTCAA
13301 CAATTGTTGT GTAGGTACTC CGCCGCCGAG GGACCTGAGC GAGTCCGCAT
13351 CGACCGGATC GGAAAACCTC TCGAGAAAGG CGTCTAACCA GTCACAGTCG
13401 CAAGGTAGGC TGAGCACCGT GGCGGGCGGC AGCGGGCGGC GGTCGGGGTT
13451 GTTTCTGGCG GAGGTGCTGC TGATGATGTA ATTAAAGTAG GCGGTCTTGA
13501 GACGGCGGAT GGTCGACAGA AGCACCATGT CCTTGGGTCC GGCCTGCTGA
13551 ATGCGCAGGC GGTCGGCCAT GCCCCAGGCT TCGTTTTGAC ATCGGCGCAG
13601 GTCTTTGTAG TAGTCTTGCA TGAGCCTTTC TACCGGCACT TCTTCTTCTC
13651 CTTCCTCTTG TCCTGCATCT CTTGCATCTA TCGCTGCGGC GGCGGCGGAG
13701 TTTGGCCGTA GGTGGCGCCC TCTTCCTCCC ATGCGTGTGA CCCCGAAGCC
13751 CCTCATCGGC TGAAGCAGGG CTAGGTCGGC GACAACGCGC TCGGCTAATA
13801 TGGCCTGCTG CACCTGCGTG AGGGTAGACT GGAAGTCATC CATGTCCACA
13851 AAGCGGTGGT ATGCGCCCGT GTTGATGGTG TAAGTGCAGT TGGCCATAAC
13901 GGACCAGTTA ACGGTCTGGT GACCCGGCTG CGAGAGCTCG GTGTACCTGA
13951 GACGCGAGTA AGCCCTCGAG TCAAATACGT AGTCGTTGCA AGTCCGCACC
14001 AGGTACTGGT ATCCCACCAA AAAGTGCGGC GGCGGCTGGC GGTAGAGGGG

14051 CCAGCGTAGG GTGGCCGGGG CTCCGGGGGC GAGATCTTCC AACATAAGGC
14101 GATGATATCC GTAGATGTAC CTGGACATCC AGGTGATGCC GGCGGCGGTG
14151 GTGGAGGCGC GCGGAAAGTC GCGGACGCGG TTCCAGATGT TGCGCAGCGG
14201 CAAAAAGTGC TCCATGGTCG GGACGCTCTG GCCGGTCAGG CGCGCGCAAT
14251 CGTTGACGCT CTAGACCGTG CAAAAGGAGA GCCTGTAAGC GGGCACTCTT
14301 CCGTGGTCTG GTGGATAAAT TCGCAAGGGT ATCATGGCGG ACGACCGGGG
14351 TTCGAGCCCC GTATCCGGCC GTCCGCCGTG ATCCATGCGG TTACCGCCCG
14401 CGTGTCGAAC CCAGGTGTGC GACGTCAGAC AACGGGGGAG TGCTCCTTTT
14451 GGCTTCCTTC CAGGCGCGGC GGCTGCTGCG CTAGCTTTTT TGGCCACTGG
14501 CCGCGCGCAG CGTAAGCGGT TAGGCTGGAA AGCGAAAGCA TTAAGTGGCT
14551 CGCTCCCTGT AGCCGGAGGG TTATTTTCCA AGGGTTGAGT CGCGGGACCC
14601 CCGGTTCGAG TCTCGGACCG GCCGGACTGC GGCGAACGGG GGTTTGCCTC
14651 CCCGTCATGC AAGACCCCGC TTGCAAATTC CTCCGGAAAC AGGGACGAGC
14701 CCCTTTTTTG CTTTTCCCAG ATGCATCCGG TGCTGCGGCA GATGCGCCCC
14751 CCTCCTCAGC AGCGGCAAGA GCAAGAGCAG CGGCAGACAT GCAGGGCACC
14801 CTCCCCTCCT CCTACCGCGT CAGGAGGGGC GACATCCGCG GTTGACGCGG
14851 CAGCAGATGG TGATTACGAA CCCCCGCGGC GCCGGGCCCG GCACTACCTG
14901 GACTTGGAGG AGGGCGAGGG CCTGGCGCGG CTAGGAGCGC CCTCTCCTGA
14951 GCGGTACCCA AGGGTGCAGC TGAAGCGTGA TACGCGTGAG GCGTACGTGC
15001 CGCGGCAGAA CCTGTTTCGC GACCGCGAGG GAGAGGAGCC CGAGGAGATG
15051 CGGGATCGAA AGTTCCACGC AGGGCGCGAG CTGCGGCATG GCCTGAATCG
15101 CGAGCGGTTG CTGCGCGAGG AGGACTTTGA GCCCGACGCG CGAACCGGGA
15151 TTAGTCCCGC GCGCGCACAC GTGGCGGCCG CCGACCTGGT AACCGCATAC
15201 GAGCAGACGG TGAACCAGGA GATTAACTTT CAAAAAAGCT TTAACAACCA
15251 CGTGCGTACG CTTGTGGCGC GCGAGGAGGT GGCTATAGGA CTGATGCATC
15301 TGTGGGACTT TGTAAGCGCG CTGGAGCAAA ACCCAAATAG CAAGCCGCTC
15351 ATGGCGCAGC TGTTCCTTAT AGTGCAGCAC AGCAGGGACA ACGAGGCATT
15401 CAGGGATGCG CTGCTAAACA TAGTAGAGCC CGAGGGCCGC TGGCTGCTCG
15451 ATTTGATAAA CATCCTGCAG AGCATAGTGG TGCAGGAGCG CAGCTTGAGC
15501 CTGGCTGACA AGGTGGCCGC CATCAACTAT TCCATGCTTA GCCTGGGCAA
15551 GTTTTACGCC CGCAAGATAT ACCATACCCC TTACGTTCCC ATAGACAAGG
15601 AGGTAAAGAT CGAGGGGTTC TACATGCGCA TGGCGCTGAA GGTGCTTACC
15651 TTGAGCGACG ACCTGGGCGT TTATCGCAAC GAGCGCATCC ACAAGGCCGT
15701 GAGCGTGAGC CGGCGGCGCG AGCTCAGCGA CCGCGAGCTG ATGCACAGCC
15751 TGCAAAGGGC CCTGGCTGGC ACGGGCAGCG GCGATAGAGA GGCCGAGTCC
15801 TACTTTGACG CGGGCGCTGA CCTGCGCTGG GCCCCAAGCC GACGCGCCCT
15851 GGAGGCAGCT GGGGCCGGAC CTGGGCTGGC GGTGGCACCC GCGCGCGCTG
15901 GCAACGTCGG CGGCGTGGAG GAATATGACG AGGACGATGA GTACGAGCCA
15951 GAGGACGGCG AGTACTAAGC GGTGATGTTT CTGATCAGAT GATGCAAGAC
16001 GCAACGGACC CGGCGGTGCG GGCGGCGCTG CAGAGCCAGC CGTCCGGCCT
16051 TAACTCCACG GACGACTGGC GCCAGGTCAT GGACCGCATC ATGTCGCTGA
16101 CTGCGCGCAA TCCTGACGCG TTCCGGCAGC AGCCGCAGGC CAACCGGCTC
16151 TCCGCAATTC TGGAAGCGGT GGTCCCGGCG CGCGCAAACC CCACGCACGA
16201 GAAGGTGCTG GCGATCGTAA ACGCGCTGGC CGAAAACAGG GCCATCCGGC
16251 CCGACGAGGC CGGCCTGGTC TACGACGCGC TGCTTCAGCG CGTGGCTCGT
16301 TACAACAGCG GCAACGTGCA GACCAACCTG GACCGGCTGG TGGGGGATGT
16351 GCGCGAGGCC GTGGCGCAGC GTGAGCGCGC GCAGCAGCAG GGCAACCTGG
16401 GCTCCATGGT TGCACTAAAC GCCTTCCTGA GTACACAGCC CGCCAACGTG
16451 CCGCGGGGAC AGGAGGACTA CACCAACTTT GTGAGCGCAC TGCGGCTAAT
16501 GGTGACTGAG ACACCGCAAA GTGAGGTGTA CCAGTCTGGG CCAGACTATT
16551 TTTTCCAGAC CAGTAGACAA GGCCTGCAGA CCGTAAACCT GAGCCAGGCT
16601 TTCAAAAACT TGCAGGGGCT GTGGGGGGTG CGGGCTCCCA CAGGCGACCG
16651 CGCGACCGTG TCTAGCTTGC TGACGCCCAA CTCGCGCCTG TTGCTGCTGC
16701 TAATAGCGCC CTTCACGGAC AGTGGCAGCG TGTCCCGGGA CACATACCTA
16751 GGTCACTTGC TGACACTGTA CCGCGAGGCC ATAGGTCAGG CGCATGTGGA
16801 CGAGCATACT TTCCAGGAGA TTACAAGTGT CAGCCGCGCG CTGGGGCAGG
16851 AGGACACGGG CAGCCTGGAG GCAACCCTAA ACTACCTGCT GACCAACCGG
16901 CGGCAGAAGA TCCCCTCGTT GCACAGTTTA AACAGCGAGG AGGAGCGCAT
16951 TTTGCGCTAC GTGCAGCAGA GCGTGAGCCT TAACCTGATG CGCGACGGGG
17001 TAACGCCCAG CGTGGCGCTG GACATGACCG CGCGCAACAT GGAACCGGGC
17051 ATGTATGCCT CAAACCGGCC GTTTATCAAC CGCCTAATGG ACTACTTGCA
17101 TCGCGCGGCC GCCGTGAACC CCGAGTATTT CACCAATGCC ATCTTGAACC
17151 CGCACTGGCT ACCGCCCCCT GGTTTCTACA CCGGGGGATT CGAGGTGCCC

17201 GAGGGTAACG ATGGATTCCT CTGGGACGAC ATAGACGACA GCGTGTTTTC
17251 CCCGCAACCG CAGACCCTGC TAGAGTTGCA ACAGCGCGAG CAGGCAGAGG
17301 CGGCGCTGCG AAAGGAAAGC TTCCGCAGGC CAAGCAGCTT GTCCGATCTA
17351 GGCGCTGCGG CCCCGCGGTC AGATGCTAGT AGCCCATTTC CAAGCTTGAT
17401 AGGGTCTCTT ACCAGCACTC GCACCACCCG CCCGCGCCTG CTGGGCGAGG
17451 AGGAGTACCT AAACAACTCG CTGCTGCAGC CGCAGCGCGA AAAAAACCTG
17501 CCTCCGGCAT TTCCCAACAA CGGGATAGAG AGCCTAGTGG ACAAGATGAG
17551 TAGATGGAAG ACGTACGCGC AGGAGCACAG GGACGTGCCA GGCCCGCGCC
17601 CGCCCACCCG TCGTCAAAGG CACGACCGTC AGCGGGGTCT GGTGTGGGAG
17651 GACGATGACT CGGCAGACGA CAGCAGCGTC CTGGATTTGG GAGGGAGTGG
17701 CAACCCGTTT GCGCACCTTC GCCCCAGGCT GGGGAGAATG TTTTAAAAAA
17751 AAAAAAGCAT GATGCAAAAT AAAAAACTCA CCAAGGCCAT GGCACCGAGC
17801 GTTGGTTTTC TTGTATTCCC CTTAGTATGC GGCGCGCGGC GATGTATGAG
17851 GAAGGTCCTC CTCCCTCCTA CGAGAGTGTG GTGAGCGCGG CGCCAGTGGC
17901 GGCGGCGCTG GGTTCTCCCT TCGATGCTCC CCTGGACCCG CCGTTTGTGC
17951 CTCCGCGGTA CCTGCGGCCT ACCGGGGGGA GAAACAGCAT CCGTTACTCT
18001 GAGTTGGCAC CCCTATTCGA CACCACCCGT GTGTACCTGG TGGACAACAA
18051 GTCAACGGAT GTGGCATCCC TGAACTACCA GAACGACCAC AGCAACTTTC
18101 TGACCACGGT CATTCAAAAC AATGACTACA GCCCGGGGGA GGCAAGCACA
18151 CAGACCATCA ATCTTGACGA CCGGTCGCAC TGGGGCGGCG ACCTGAAAAC
18201 CATCCTGCAT ACCAACATGC CAAATGTGAA CGAGTTCATG TTTACCAATA
18251 AGTTTAAGGC GCGGGTGATG GTGTCGCGCT TGCCTACTAA GGACAATCAG
18301 GTGGAGCTGA AATACGAGTG GGTGGAGTTC ACGCTGCCCG AGGGCAACTA
18351 CTCCGAGACC ATGACCATAG ACCTTATGAA CAACGCGATC GTGGAGCACT
18401 ACTTGAAAGT GGGCAGACAG AACGGGGTTC TGGAAAGCGA CATCGGGGTA
18451 AAGTTTGACA CCCGCAACTT CAGACTGGGG TTTGACCCCG TCACTGGTCT
18501 TGTCATGCCT GGGGTATATA CAAACGAAGC CTTCCATCCA GACATCATTT
18551 TGCTGCCAGG ATGCGGGGTG GACTTCACCC ACAGCCGCCT GAGCAACTTG
18601 TTGGGCATCC GCAAGCGGCA ACCCTTCCAG GAGGGCTTTA GGATCACCTA
18651 CGATGATCTG GAGGGTGGTA ACATTCCCGC ACTGTTGGAT GTGGACGCCT
18701 ACCAGGCGAG CTTGAAAGAT GACACCGAAC AGGGCGGGGG TGGCGCAGGC
18751 GGCAGCAACA GCAGTGGCAG CGGCGCGGAA GAGAACTCCA ACGCGGCAGC
18801 CGCGGCAATG CAGCCGGTGG AGGACATGAA CGATCATGCC ATTCGCGGCG
18851 ACACCTTTGC CACACGGGCT GAGGAGAAGC GCGCTGAGGC CGAAGCAGCG
18901 GCCGAAGCTG CCGCCCCCGC TGCGCAACCC GAGGTCGAGA AGCCTCAGAA
18951 GAAACCGGTG ATCAAACCCC TGACAGAGGA CAGCAAGAAA CGCAGTTACA
19001 ACCTAATAAG CAATGACAGC ACCTTCACCC AGTACCGCAG CTGGTACCTT
19051 GCATACAACT ACGGCGACCC TCAGACCGGA ATCCGCTCAT GGACCCTGCT
19101 TTGCACTCCT GACGTAACCT GCGGCTCGGA GCAGGTCTAC TGGTCGTTGC
19151 CAGACATGAT GCAAGACCCC GTGACCTTCC GCTCCACGCG CCAGATCAGC
19201 AACTTTCCGG TGGTGGGCGC CGAGCTGTTG CCCGTGCACT CCAAGAGCTT
19251 CTACAACGAC CAGGCCGTCT ACTCCCAACT CATCCGCCAG TTTACCTCTC
19301 TGACCCACGT GTTCAATCGC TTTCCCGAGA ACCAGATTTT GGCGCGCCCG
19351 CCAGCCCCCA CCATCACCAC CGTCAGTGAA AACGTTCCTG CTCTCACAGA
19401 TCACGGGACG CTACCGCTGC GCAACAGCAT CGGAGGAGTC CAGCGAGTGA
19451 CCATTACTGA CGCCAGACGC CGCACCTGCC CCTACGTTTA CAAGGCCCTG
19501 GGCATAGTCT CGCCGCGCGT CCTATCGAGC CGCACTTTTT GAGCAAGCAT
19551 GTCCATCCTT ATATCGCCCA GCAATAACAC AGGCTGGGGC CTGCGCTTCC
19601 CAAGCAAGAT GTTTGGCGGG GCCAAGAAGC GCTCCGACCA ACACCCAGTG
19651 CGCGTGCGCG GGCACTACCG CGCGCCCTGG GGCGCGCACA AACGCGGCCG
19701 CACTGGGCGC ACCACCGTCG ATGACGCCAT CGACGCGGTG GTGGAGGAGG
19751 CGCGCAACTA CACGCCCACG CCGCCACCAG TGTCCACAGT GGACGCGGCC
19801 ATTCAGACCG TGGTGCGCGG AGCCCGGCGC TATGCTAAAA TGAAGAGACG
19851 GCGGAGGCGC GTAGCACGTC GCCACCGCCG CCGACCCGGC ACTGCCGCCC
19901 AACGCGCGGC GGCGGCCCTG CTTAACCGCG CACGTCGCAC CGGCCGACGG
19951 GCGGCCATGC GGGCCGCTCG AAGGCTGGCC GCGGGTATTG TCACTGTGCC
20001 CCCCAGGTCC AGGCGACGAG CGGCCGCCGC AGCAGCCGCG GCCATTAGTG
20051 CTATGACTCA GGGTCGCAGG GGCAACGTGT ATTGGGTGCG CGACTCGGTT
20101 AGCGGCCTGC GCGTGCCCGT GCGCACCCGC CCCCCGCGCA ACTAGATTGC
20151 AAGAAAAAAC TACTTAGACT CGTACTGTTG TATGTATCCA GCGGCGGCGG
20201 CGCGCAACGA AGCTATGTCC AAGCGCAAAA TCAAAGAAGA GATGCTCCAG
20251 GTCATCGCGC CGGAGATCTA TGGCCCCCCG AAGAAGGAAG AGCAGGATTA
20301 CAAGCCCCGA AAGCTAAAGC GGGTCAAAAA GAAAAAGAAA GATGATGATG

20351 ATGAACTTGA CGACGAGGTG GAACTGCTGC ACGCTACCGC GCCCAGGCGA
20401 CGGGTACAGT GGAAAGGTCG ACGCGTAAAA CGTGTTTTGC GACCCGGCAC
20451 CACCGTAGTC TTTACGCCCG GTGAGCGCTC CACCCGCACC TACAAGCGCG
20501 TGTATGATGA GGTGTACGGC GACGAGGACC TGCTTGAGCA GGCCAACGAG
20551 CGCCTCGGGG AGTTTGCCTA CGGAAAGCGG CATAAGGACA TGCTGGCGTT
20601 GCCGCTGGAC GAGGGCAACC CAACACCTAG CCTAAAGCCC GTAACACTGC
20651 AGCAGGTGCT GCCCGCGCTT GCACCGTCCG AAGAAAAGCG CGGCCTAAAG
20701 CGCGAGTCTG GTGACTTGGC ACCCACCGTG CAGCTGATGG TACCCAAGCG
20751 CCAGCGACTG GAAGATGTCT TGGAAAAAAT GACCGTGGAA CCTGGGCTGG
20801 AGCCCGAGGT CCGCGTGCGG CCAATCAAGC AGGTGGCGCC GGGACTGGGC
20851 GTGCAGACCG TGGACGTTCA GATACCCACT ACCAGTAGCA CCAGTATTGC
20901 CACCGCCACA GAGGGCATGG AGACACAAAC GTCCCCGGTT GCCTCAGCGG
20951 TGGCGGATGC CGCGGTGCAG GCGGTCGCTG CGGCCGCGTC CAAGACCTCT
21001 ACGGAGGTGC AAACGGACCC GTGGATGTTT CGCGTTTCAG CCCCCCGGCG
21051 CCCGCGCGGT TCGAGGAAGT ACGGCGCCGC CAGCGCGCTA CTGCCCGAAT
21101 ATGCCCTACA TCCTTCCATT GCGCCTACCC CCGGCTATCG TGGCTACACC
21151 TACCGCCCCA GAAGACGAGC AACTACCCGA CGCCGAACCA CCACTGGAAC
21201 CCGCCGCCGC CGTCGCCGTC GCCAGCCCGT GCTGGCCCCG ATTTCCGTGC
21251 GCAGGGTGGC TCGCGAAGGA GGCAGGACCC TGGTGCTGCC AACAGCGCGC
21301 TACCACCCCA GCATCGTTTA AAAGCCGGTC TTTGTGGTTC TTGCAGATAT
21351 GGCCCTCACC TGCCGCCTCC GTTTCCCGGT GCCGGGATTC CGAGGAAGAA
21401 TGCACCGTAG GAGGGGCATG GCCGGCCACG GCCTGACGGG CGGCATGCGT
21451 CGTGCGCACC ACCGGCGGCG GCGCGCGTCG CACCGTCGCA TGCGCGGCGG
21501 TATCCTGCCC CTCCTTATTC CACTGATCGC CGCGGCGATT GGCGCCGTGC
21551 CCGGAATTGC ATCCGTGGCC TTGCAGGCGC AGAGACACTG ATTAAAAACA
21601 AGTTGCATGT GGAAAAATCA AAATAAAAAG TCTGGACTCT CACGCTCGCT
21651 TGGTCCTGTA ACTATTTTGT AGAATGGAAG ACATCAACTT TGCGTCTCTG
21701 GCCCCGCGAC ACGGCTCGCG CCCGTTCATG GGAAACTGGC AAGATATCGG
21751 CACCAGCAAT ATGAGCGGTG GCGCCTTCAG CTGGGGCTCG CTGTGGAGCG
21801 GCATTAAAAA TTTCGGTTCC ACCGTTAAGA ACTATGGCAG CAAGGCCTGG
21851 AACAGCAGCA CAGGCCAGAT GCTGAGGGAT AAGTTGAAAG AGCAAAATTT
21901 CCAACAAAAG GTGGTAGATG GCCTGGCCTC TGGCATTAGC GGGGTGGTGG
21951 ACCTGGCCAA CCAGGCAGTG CAAAATAAGA TTAACAGTAA GCTTGATCCC
22001 CGCCCTCCCG TAGAGGAGCC TCCACCGGCC GTGGAGACAG TGTCTCCAGA
22051 GGGGCGTGGC GAAAAGCGTC CGCGCCCCGA CAGGGAAGAA ACTCTGGTGA
22101 CGCAAATAGA CGAGCCTCCC TCGTACGAGG AGGCACTAAA GCAAGGCCTG
22151 CCCACCACCC GTCCCATCGC GCCCATGGCT ACCGGAGTGC TGGGCCAGCA
22201 CACACCCGTA ACGCTGGACC TGCCTCCCCC CGCCGACACC CAGCAGAAAC
22251 CTGTGCTGCC AGGCCCGACC GCCGTTGTTG TAACCCGTCC TAGCCGCGCG
22301 TCCCTGCGCC GCGCCGCCAG CGGTCCGCGA TCGTTGCGGC CCGTAGCCAG
22351 TGGCAACTGG CAAAGCACAC TGAACAGCAT CGTGGGTCTG GGGGTGCAAT
22401 CCCTGAAGCG CCGACGATGC TTCTGAATAG CTAACGTGTC GTATGTGTGT
22451 CATGTATGCG TCCATGTCGC CGCCAGAGGA GCTGCTGAGC CGCCGCGCGC
22501 CCGCTTTCCA AGATGGCTAC CCCTTCGATG ATGCCGCAGT GGTCTTACAT
22551 GCACATCTCG GGCCAGGACG CCTCGGAGTA CCTGAGCCCC GGGCTGGTGC
22601 AGTTTGCCCG CGCCACCGAG ACGTACTTCA GCCTGAATAA CAAGTTTAGA
22651 AACCCCACGG TGGCGCCTAC GCACGACGTG ACCACAGACC GGTCCCAGCG
22701 TTTGACGCTG CGGTTCATCC CTGTGGACCG TGAGGATACT GCGTACTCGT
22751 ACAAGGCGCG GTTCACCCTA GCTGTGGGTG ATAACCGTGT GCTGGACATG
22801 GCTTCCACGT ACTTTGACAT CCGCGGCGTG CTGGACAGGG GCCCTACTTT
22851 TAAGCCCTAC TCTGGCACTG CCTACAACGC CCTGGCTCCC AAGGGTGCCC
22901 CAAATCCTTG CGAATGGGAT GAAGCTGCTA CTGCTCTTGA AATAAACCTA
22951 GAAGAAGAGG ACGATGACAA CGAAGACGAA GTAGACGAGC AAGCTGAGCA
23001 GCAAAAAACT CACGTATTTG GGCAGGCGCC TTATTCTGGT ATAAATATTA
23051 CAAAGGAGGG TATTCAAATA GGTGTCGAAG GTCAAACACC TAAATATGCC
23101 GATAAAACAT TTCAACCTGA ACCTCAAATA GGAGAATCTC AGTGGTACGA
23151 AACTGAAATT AATCATGCAG CTGGGAGAGT CCTTAAAAAG ACTACCCCAA
23201 TGAAACCATG TTACGGTTCA TATGCAAAAC CCACAAATGA AAATGGAGGG
23251 CAAGGCATTC TTGTAAAGCA ACAAAATGGA AAGCTAGAAA GTCAAGTGGA
23301 AATGCAATTT TTCTCAACTA CTGAGGCGAC CGCAGGCAAT GGTGATAACT
23351 TGACTCCTAA AGTGGTATTG TACAGTGAAG ATGTAGATAT AGAAACCCCA
23401 GACACTCATA TTTCTTACAT GCCCACTATT AAGGAAGGTA ACTCACGAGA
23451 ACTAATGGGC CAACAATCTA TGCCCAACAG GCCTAATTAC ATTGCTTTTA

23501 GGGACAATTT TATTGGTCTA ATGTATTACA ACAGCACGGG TAATATGGGT
23551 GTTCTGGCGG GCCAAGCATC GCAGTTGAAT GCTGTTGTAG ATTTGCAAGA
23601 CAGAAACACA GAGCTTTCAT ACCAGCTTTT GCTTGATTCC ATTGGTGATA
23651 GAACCAGGTA CTTTTCTATG TGGAATCAGG CTGTTGACAG CTATGATCCA
23701 GATGTTAGAA TTATTGAAAA TCATGGAACT GAAGATGAAC TTCCAAATTA
23751 CTGCTTTCCA CTGGGAGGTG TGATTAATAC AGAGACTCTT ACCAAGGTAA
23801 AACCTAAAAC AGGTCAGGAA AATGGATGGG AAAAAGATGC TACAGAATTT
23851 TCAGATAAAA ATGAAATAAG AGTTGGAAAT AATTTTGCCA TGGAAATCAA
23901 TCTAAATGCC AACCTGTGGA GAAATTTCCT GTACTCCAAC ATAGCGCTGT
23951 ATTTGCCCGA CAAGCTAAAG TACAGTCCTT CCAACGTAAA AATTTCTGAT
24001 AACCCAAACA CCTACGACTA CATGAACAAG CGAGTGGTGG CTCCCGGGTT
24051 AGTGGACTGC TACATTAACC TTGGAGCACG CTGGTCCCTT GACTATATGG
24101 ACAACGTCAA CCCATTTAAC CACCACCGCA ATGCTGGCCT GCGCTACCGC
24151 TCAATGTTGC TGGGCAATGG TCGCTATGTG CCCTTCCACA TCCAGGTGCC
24201 TCAGAAGTTC TTTGCCATTA AAAACCTCCT TCTCCTGCCG GGCTCATACA
24251 CCTACGAGTG GAACTTCAGG AAGGATGTTA ACATGGTTCT GCAGAGCTCC
24301 CTAGGAAATG ACCTAAGGGT TGACGGAGCC AGCATTAAGT TTGATAGCAT
24351 TTGCCTTTAC GCCACCTTCT TCCCCATGGC CCACAACACC GCCTCCACGC
24401 TTGAGGCCAT GCTTAGAAAC GACACCAACG ACCAGTCCTT TAACGACTAT
24451 CTCTCCGCCG CCAACATGCT CTACCCTATA CCCGCCAACG CTACCAACGT
24501 GCCCATATCC ATCCCCTCCC GCAACTGGGC GGCTTTCCGC GGCTGGGCCT
24551 TCACGCGCCT TAAGACTAAG GAAACCCCAT CACTGGGCTC GGGCTACGAC
24601 CCTTATTACA CCTACTCTGG CTCTATACCC TACCTAGATG GAACCTTTTA
24651 CCTCAACCAC ACCTTTAAGA AGGTGGCCAT TACCTTTGAC TCTTCTGTCA
24701 GCTGGCCTGG CAATGACCGC CTGCTTACCC CCAACGAGTT TGAAATTAAG
24751 CGCTCAGTTG ACGGGGAGGG TTACAACGTT GCCCAGTGTA ACATGACCAA
24801 AGACTGGTTC CTGGTACAAA TGCTAGCTAA CTACAACATT GGCTACCAGG
24851 GCTTCTATAT CCCAGAGAGC TACAAGGACC GCATGTACTC CTTCTTTAGA
24901 AACTTCCAGC CCATGAGCCG TCAGGTGGTG GATGATACTA AATACAAGGA
24951 CTACCAACAG GTGGGCATCC TACACCAACA CAACAACTCT GGATTTGTTG
25001 GCTACCTTGC CCCCACCATG CGCGAAGGAC AGGCCTACCC TGCTAACTTC
25051 CCCTATCCGC TTATAGGCAA GACCGCAGTT GACAGCATTA CCCAGAAAAA
25101 GTTTCTTTGC GATCGCACCC TTTGGCGCAT CCCATTCTCC AGTAACTTTA
25151 TGTCCATGGG CGCACTCACA GACCTGGGCC AAAACCTTCT CTACGCCAAC
25201 TCCGCCCACG CGCTAGACAT GACTTTTGAG GTGGATCCCA TGGACGAGCC
25251 CACCCTTCTT TATGTTTTGT TTGAAGTCTT TGACGTGGTC CGTGTGCACC
25301 GGCCGCACCG CGGCGTCATC GAAACCGTGT ACCTGCGCAC GCCCTTCTCG
25351 GCCGGCAACG CCACAACATA AAGAAGCAAG CAACATCAAC AACAGCTGCC
25401 GCCATGGGCT CCAGTGAGCA GGAACTGAAA GCCATTGTCA AAGATCTTGG
25451 TTGTGGGCCA TATTTTTTGG GCACCTATGA CAAGCGCTTT CCAGGCTTTG
25501 TTTCTCCACA CAAGCTCGCC TGCGCCATAG TCAATACGGC CGGTCGCGAG
25551 ACTGGGGGCG TACACTGGAT GGCCTTTGCC TGGAACCCGC ACTCAAAAAC
25601 ATGCTACCTC TTTGAGCCCT TTGGCTTTTC TGACCAGCGA CTCAAGCAGG
25651 TTTACCAGTT TGAGTACGAG TCACTCCTGC GCCGTAGCGC CATTGCTTCT
25701 TCCCCCGACC GCTGTATAAC GCTGGAAAAG TCCACCCAAA GCGTACAGGG
25751 GCCCAACTCG GCCGCCTGTG GACTATTCTG CTGCATGTTT CTCCACGCCT
25801 TTGCCAACTG GCCCCAAACT CCCATGGATC ACAACCCCAC CATGAACCTT
25851 ATTACCGGGG TACCCAACTC CATGCTCAAC AGTCCCCAGG TACAGCCCAC
25901 CCTGCGTCGC AACCAGGAAC AGCTCTACAG CTTCCTGGAG CGCCACTCGC
25951 CCTACTTCCG CAGCCACAGT GCGCAGATTA GGAGCGCCAC TTCTTTTTGT
26001 CACTTGAAAA ACATGTAAAA ATAATGTACT AGAGACACTT TCAATAAAGG
26051 CAAATGCTTT TATTTGTACA CTCTCGGGTG ATTATTTACC CCCACCCTTG
26101 CCGTCTGCGC CGTTTAAAAA TCAAAGGGGT TCTGCCGCGC ATCGCTATGC
26151 GCCACTGGCA GGGACACGTT GCGATACTGG TGTTTAGTGC TCCACTTAAA
26201 CTCAGGCACA ACCATCCGCG GCAGCTCGGT GAAGTTTTCA CTCCACAGGC
26251 TGCGCACCAT CACCAACGCG TTTAGCAGGT CGGGCGCCGA TATCTTGAAG
26301 TCGCAGTTGG GGCCTCCGCC CTGCGCGCGC GAGTTGCGAT ACACAGGGTT
26351 GCAGCACTGG AACACTATCA GCGCCGGGTG GTGCACGCTG GCCAGCACGC
26401 TCTTGTCGGA GATCAGATCC GCGTCCAGGT CCTCCGCGTT GCTCAGGGCG
26451 AACGGAGTCA ACTTTGGTAG CTGCCTTCCC AAAAAGGGCG CGTGCCCAGG
26501 CTTTGAGTTG CACTCGCACC GTAGTGGCAT CAAAAGGTGA CCGTGCCCGG
26551 TCTGGGCGTT AGGATACAGC GCCTGCATAA AAGCCTTGAT CTGCTTAAAA
26601 GCCACCTGAG CCTTTGCGCC TTCAGAGAAG AACATGCCGC AAGACTTGCC

26651 GGAAAACTGA TTGGCCGGAC AGGCCGCGTC GTGCACGCAG CACCTTGCGT
26701 CGGTGTTGGA GATCTGCACC ACATTTCGGC CCCACCGGTT CTTCACGATC
26751 TTGGCCTTGC TAGACTGCTC CTTCAGCGCG CGCTGCCCGT TTTCGCTCGT
26801 CACATCCATT TCAATCACGT GCTCCTTATT TATCATAATG CTTCCGTGTA
26851 GACACTTAAG CTCGCCTTCG ATCTCAGCGC AGCGGTGCAG CCACAACGCG
26901 CAGCCCGTGG GCTCGTGATG CTTGTAGGTC ACCTCTGCAA ACGACTGCAG
26951 GTACGCCTGC AGGAATCGCC CCATCATCGT CACAAAGGTC TTGTTGCTGG
27001 TGAAGGTCAG CTGCAACCCG CGGTGCTCCT CGTTCAGCCA GGTCTTGCAT
27051 ACGGCCGCCA GAGCTTCCAC TTGGTCAGGC AGTAGTTTGA AGTTCGCCTT
27101 TAGATCGTTA TCCACGTGGT ACTTGTCCAT CAGCGCGCGC GCAGCCTCCA
27151 TGCCCTTCTC CCACGCAGAC ACGATCGGCA CACTCAGCGG GTTCATCACC
27201 GTAATTTCAC TTTCCGCTTC GCTGGGCTCT TCCTCTTCCT CTTGCGTCCG
27251 CATACCACGC GCCACTGGGT CGTCTTCATT CAGCCGCCGC ACTGTGCGCT
27301 TACCTCCTTT GCCATGCTTG ATTAGCACCG GTGGGTTGCT GAAACCCACC
27351 ATTTGTAGCG CCACATCTTC TCTTTCTTCC TCGCTGTCCA CGATTACCTC
27401 TGGTGATGGC GGGCGCTCGG GCTTGGGAGA AGGGCGCTTC TTTTTCTTCT
27451 TGGGCGCAAT GGCCAAATCC GCCGCCGAGG TCGATGGCCG CGGGCTGGGT
27501 GTGCGCGGCA CCAGCGCGTC TTGTGATGAG TCTTCCTCGT CCTCGGACTC
27551 GATACGCCGC CTCATCCGCT TTTTTGGGGG CGCCCGGGGA GGCGGCGGCG
27601 ACGGGGACGG GGACGACACG TCCTCCATGG TTGGGGGACG TCGCGCCGCA
27651 CCGCGTCCGC GCTCGGGGGT GGTTTCGCGC TGCTCCTCTT CCCGACTGGC
27701 CATTTCCTTC TCCTATAGGC AGAAAAAGAT CATGGAGTCA GTCGAGAAGA
27751 AGGACAGCCT AACCGCCCCC TCTGAGTTCG CCACCACCGC CTCCACCGAT
27801 GCCGCCAACG CGCCTACCAC CTTCCCCGTC GAGGCACCCC CGCTTGAGGA
27851 GGAGGAAGTG ATTATCGAGC AGGACCCAGG TTTTGTAAGC GAAGACGACG
27901 AGGACCGCTC AGTACCAACA GAGGATAAAA AGCAAGACCA GGACAACGCA
27951 GAGGCAAACG AGGAACAAGT CGGGCGGGGG GACGAAAGGC ATGGCGACTA
28001 CCTAGATGTG GGAGACGACG TGCTGTTGAA GCATCTGCAG CGCCAGTGCG
28051 CCATTATCTG CGACGCGTTG CAAGAGCGCA GCGATGTGCC CCTCGCCATA
28101 GCGGATGTCA GCCTTGCCTA CGAACGCCAC CTATTCTCAC CGCGCGTACC
28151 CCCCAAACGC CAAGAAAACG GCACATGCGA GCCCAACCCG CGCCTCAACT
28201 TCTACCCCGT ATTTGCCGTG CCAGAGGTGC TTGCCACCTA TCACATCTTT
28251 TTCCAAAACT GCAAGATACC CCTATCCTGC CGTGCCAACC GCAGCCGAGC
28301 GGACAAGCAG CTGGCCTTGC GGCAGGGCGC TGTCATACCT GATATCGCCT
28351 CGCTCAACGA AGTGCCAAAA ATCTTTGAGG GTCTTGGACG CGACGAGAAG
28401 CGCGCGGCAA ACGCTCTGCA ACAGGAAAAC AGCGAAAATG AAAGTCACTC
28451 TGGAGTGTTG GTGGAACTCG AGGGTGACAA CGCGCGCCTA GCCGTACTAA
28501 AACGCAGCAT CGAGGTCACC CACTTTGCCT ACCCGGCACT TAACCTACCC
28551 CCCAAGGTCA TGAGCACAGT CATGAGTGAG CTGATCGTGC GCCGTGCGCA
28601 GCCCCTGGAG AGGGATGCAA ATTTGCAAGA ACAAACAGAG GAGGGCCTAC
28651 CCGCAGTTGG CGACGAGCAG CTAGCGCGCT GGCTTCAAAC GCGCGAGCCT
28701 GCCGACTTGG AGGAGCGACG CAAACTAATG ATGGCCGCAG TGCTCGTTAC
28751 CGTGGAGCTT GAGTGCATGC AGCGGTTCTT TGCTGACCCG GAGATGCAGC
28801 GCAAGCTAGA GGAAACATTG CACTACACCT TTCGACAGGG CTACGTACGC
28851 CAGGCCTGCA AGATCTCCAA CGTGGAGCTC TGCAACCTGG TCTCCTACCT
28901 TGGAATTTTG CACGAAAACC GCCTTGGGCA AAACGTGCTT CATTCCACGC
28951 TCAAGGGCGA GGCGCGCCGC GACTACGTCC GCGACTGCGT TTACTTATTT
29001 CTATGCTACA CCTGGCAGAC GGCCATGGGC GTTTGGCAGC AGTGCTTGGA
29051 GGAGTGCAAC CTCAAGGAGC TGCAGAAACT GCTAAAGCAA AACTTGAAGG
29101 ACCTATGGAC GGCCTTCAAC GAGCGCTCCG TGGCCGCGCA CCTGGCGGAC
29151 ATCATTTTCC CCGAACGCCT GCTTAAAACC CTGCAACAGG GTCTGCCAGA
29201 CTTCACCAGT CAAAGCATGT TGCAGAACTT TAGGAACTTT ATCCTAGAGC
29251 GCTCAGGAAT CTTGCCCGCC ACCTGCTGTG CACTTCCTAG CGACTTTGTG
29301 CCCATTAAGT ACCGCGAATG CCCTCCGCCG CTTTGGGGCC ACTGCTACCT
29351 TCTGCAGCTA GCCAACTACC TTGCCTACCA CTCTGACATA ATGGAAGACG
29401 TGAGCGGTGA CGGTCTACTG GAGTGTCACT GTCGCTGCAA CCTATGCACC
29451 CCGCACCGCT CCCTGGTTTG CAATTCGCAG CTGCTTAACG AAAGTCAAAT
29501 TATCGGTACC TTTGAGCTGC AGGGTCCCTC GCCTGACGAA AAGTCCGCGG
29551 CTCCGGGGTT GAAACTCACT CCGGGGCTGT GGACGTCGGC TTACCTTCGC
29601 AAATTTGTAC CTGAGGACTA CCACGCCCAC GAGATTAGGT TCTACGAAGA
29651 CCAATCCCGC CCGCCAAATG CGGAGCTTAC CGCCTGCGTC ATTACCCAGG
29701 GCCACATTCT TGGCCAATTG CAAGCCATCA ACAAAGCCCG CCAAGAGTTT
29751 CTGCTACGAA AGGGACGGGG GGTTTACTTG GACCCCCAGT CCGGCGAGGA

29801 GCTCAACCCA ATCCCCCCGC CGCCGCAGCC CTATCAGCAG CAGCCGCGGG
29851 CCCTTGCTTC CCAGGATGGC ACCCAAAAAG AAGCTGCAGC TGCCGCCGCC
29901 ACCCACGGAC GAGGAGGAAT ACTGGGACAG TCAGGCAGAG GAGGTTTTGG
29951 ACGAGGAGGA GGAGGACATG ATGGAAGACT GGGAGAGCCT AGACGAGGAA
30001 GCTTCCGAGG TCGAAGAGGT GTCAGACGAA ACACCGTCAC CCTCGGTCGC
30051 ATTCCCCTCG CCGGCGCCCC AGAAATCGGC AACCGGTTCC AGCATGGCTA
30101 CAACCTCCGC TCCTCAGGCG CCGCCGGCAC TGCCCGTTCG CCGACCCAAC
30151 CGTAGATGGG ACACCACTGG AACCAGGGCC GGTAAGTCCA AGCAGCCGCC
30201 GCCGTTAGCC CAAGAGCAAC AACAGCGCCA AGGCTACCGC TCATGGCGCG
30251 GGCACAAGAA CGCCATAGTT GCTTGCTTGC AAGACTGTGG GGGCAACATC
30301 TCCTTCGCCC GCCGCTTTCT TCTCTACCAT CACGGCGTGG CCTTCCCCCG
30351 TAACATCCTG CATTACTACC GTCATCTCTA CAGCCCATAC TGCACCGGCG
30401 GCAGCGGCAG CGGCAGCAAC AGCAGCGGCC ACACAGAAGC AAAGGCGACC
30451 GGATAGCAAG ACTCTGACAA AGCCCAAGAA ATCCACAGCG GCGGCAGCAG
30501 CAGGAGGAGG AGCGCTGCGT CTGGCGCCCA ACGAACCCGT ATCGACCCGC
30551 GAGCTTAGAA ACAGGATTTT TCCCACTCTG TATGCTATAT TTCAACAGAG
30601 CAGGGGCCAA GAACAAGAGC TGAAAATAAA AAACAGGTCT CTGCGATCCC
30651 TCACCCGCAG CTGCCTGTAT CACAAAAGCG AAGATCAGCT TCGGCGCACG
30701 CTGGAAGACG CGGAGGCTCT CTTCAGTAAA TACTGCGCGC TGACTCTTAA
30751 GGACTAGTTT CGCGCCCTTT CTCAAATTTA AGCGCGAAAA CTACGTCATC
30801 TCCAGCGGCC ACACCCGGCG CCAGCACCTG TCGTCAGCGC CATTATGAGC
30851 AAGGAAATTC CCACGCCCTA CATGTGGAGT TACCAGCCAC AAATGGGACT
30901 TGCGGCTGGA GCTGCCCAAG ACTACTCAAC CCGAATAAAC TACATGAGCG
30951 CGGGACCCCA CATGATATCC CGGGTCAACG GAATCCGCGC CCACCGAAAC
31001 CGAATTCTCT TGGAACAGGC GGCTATTACC ACCACACCTC GTAATAACCT
31051 TAATCCCCGT AGTTGGCCCG CTGCCCTGGT GTACCAGGAA AGTCCCGCTC
31101 CCACCACTGT GGTACTTCCC AGAGACGCCC AGGCCGAAGT TCAGATGACT
31151 AACTCAGGGG CGCAGCTTGC GGGCGGCTTT CGTCACAGGG TGCGGTCGCC
31201 CGGGCAGGGT ATAACTCACC TGACAATCAG AGGGCGAGGT ATTCAGCTCA
31251 ACGACGAGTC GGTGAGCTCC TCGCTTGGTC TCCGTCCGGA CGGGACATTT
31301 CAGATCGGCG GCGCCGGCCG TCCTTCATTC ACGCCTCGTC AGGCAATCCT
31351 AACTCTGCAG ACCTCGTCCT CTGAGCCGCG CTCTGGAGGC ATTGGAACTC
31401 TGCAATTTAT TGAGGAGTTT GTGCCATCGG TCTACTTTAA CCCCTTCTCG
31451 GGACCTCCCG GCCACTATCC GGATCAATTT ATTCCTAACT TTGACGCGGT
31501 AAAGGACTCG GCGGACGGCT ACGACTGAAT GTTAAGTGGA GAGGCAGAGC
31551 AACTGCGCCT GAAACACCTG GTCCACTGTC GCCGCCACAA GTGCTTTGCC
31601 CGCGACTCCG GTGAGTTTTG CTACTTTGAA TTGCCCGAGG ATCATATCGA
31651 GGGCCCGGCG CACGGCGTCC GGCTTACCGC CCAGGGAGAG CTTGCCCGTA
31701 GCCTGATTCG GGAGTTTACC CAGCGCCCCC TGCTAGTTGA GCGGGACAGG
31751 GGACCCTGTG TTCTCACTGT GATTTGCAAC TGTCCTAACC TTGGATTACA
31801 TCAAGATCTT TGTTGCCATC TCTGTGCTGA GTATAATAAA TACAGAAATT
31851 AAAATATACT GGGGCTCCTA TCGCCATCCT GTAAACGCCA CCGTCTTCAC
31901 CCGCCCAAGC AAACCAAGGC GAACCTTACC TGGTACTTTT AACATCTCTC
31951 CCTCTGTGAT TTACAACAGT TTCAACCCAG ACGGAGTGAG TCTACGAGAG
32001 AACCTCTCCG AGCTCAGCTA CTCCATCAGA AAAAACACCA CCCTCCTTAC
32051 CTGCCGGGAA CGTACGAGTG CGTCACCGGC CGCTGCACCA CACCTACCGC
32101 CTGACCGTAA ACCAGACTTT TTCCGGACAG ACCTCAATAA CTCTGTTTAC
32151 CAGAACAGGA GGTGAGCTTA GAAAACCCTT AGGGTATTAG GCCAAAGGCG
32201 CAGCTACTGT GGGGTTTATG AACAATTCAA GCAACTCTAC GGGCTATTCT
32251 AATTCAGGTT TCTCTAGAAA TGGACGGAAT TATTACAGAG CAGCGCCTGC
32301 TAGAAAGACG CAGGGCAGCG GCCGAGCAAC AGCGCATGAA TCAAGAGCTC
32351 CAAGACATGG TTAACTTGCA CCAGTGCAAA AGGGGTATCT TTTGTCTGGT
32401 AAAGCAGGCC AAAGTCACCT ACGACAGTAA TACCACCGGA CACCGCCTTA
32451 GCTACAAGTT GCCAACCAAG CGTCAGAAAT TGGTGGTCAT GGTGGGAGAA
32501 AAGCCCATTA CCATAACTCA GCACTCGGTA GAAACCGAAG GCTGCATTCA
32551 CTCACCTTGT CAAGGACCTG AGGATCTCTG CACCCTTATT AAGACCCTGT
32601 GCGGTCTCAA AGATCTTATT CCCTTTAACT AATAAAAAAA AATAATAAAG
32651 CATCACTTAC TTAAAATCAG TTAGCAAATT TCTGTCCAGT TTATTCAGCA
32701 GCACCTCCTT GCCCTCCTCC CAGCTCTGGT ATTGCAGCTT CCTCCTGGCT
32751 GCAAACTTTC TCCACAATCT AAATGGAATG TCAGTTTCCT CCTGTTCCTG
32801 TCCATCCGCA CCCACTATCT TCATGTTGTT GCAGATGAAG CGCGCAAGAC
32851 CGTCTGAAGA TACCTTCAAC CCCGTGTATC CATATGACAC GGAAACCGGT
32901 CCTCCAACTG TGCCTTTTCT TACTCCTCCC TTTGTATCCC CCAATGGGTT

32951 TCAAGAGAGT CCCCCTGGGG TACTCTCTTT GCGCCTATCC GAACCTCTAG
33001 TTACCTCCAA TGGCATGCTT GCGCTCAAAA TGGGCAACGG CCTCTCTCTG
33051 GACGAGGCCG GCAACCTTAC CTCCCAAAAT GTAACCACTG TGAGCCCACC
33101 TCTCAAAAAA ACCAAGTCAA ACATAAACCT GGAAATATCT GCACCCCTCA
33151 CAGTTACCTC AGAAGCCCTA ACTGTGGCTG CCGCCGCACC TCTAATGGTC
33201 GCGGGCAACA CACTCACCAT GCAATCACAG GCCCCGCTAA CCGTGCACGA
33251 CTCCAAACTT AGCATTGCCA CCCAAGGACC CCTCACAGTG TCAGAAGGAA
33301 AGCTAGCCCT GCAAACATCA GGCCCCCTCA CCACCACCGA TAGCAGTACC
33351 CTTACTATCA CTGCCTCACC CCCTCTAACT ACTGCCACTG GTAGCTTGGG
33401 CATTGACTTG AAAGAGCCCA TTTATACACA AAATGGAAAA CTAGGACTAA
33451 AGTACGGGGC TCCTTTGCAT GTAACAGACG ACCTAAACAC TTTGACCGTA
33501 GCAACTGGTC CAGGTGTGAC TATTAATAAT ACTTCCTTGC AAACTAAAGT
33551 TACTGGAGCC TTGGGTTTTG ATTCACAAGG CAATATGCAA CTTAATGTAG
33601 CAGGAGGACT AAGGATTGAT TCTCAAAACA GACGCCTTAT ACTTGATGTT
33651 AGTTATCCGT TTGATGCTCA AAACCAACTA AATCTAAGAC TAGGACAGGG
33701 CCCTCTTTTT ATAAACTCAG CCCACAACTT GGATATTAAC TACAACAAAG
33751 GCCTTTACTT GTTTACAGCT TCAAACAATT CCAAAAAGCT TGAGGTTAAC
33801 CTAAGCACTG CCAAGGGGTT GATGTTTGAC GCTACAGCCA TAGCCATTAA
33851 TGCAGGAGAT GGGCTTGAAT TTGGTTCACC TAATGCACCA AACACAAATC
33901 CCCTCAAAAC AAAAATTGGC CATGGCCTAG AATTTGATTC AAACAAGGCT
33951 ATGGTTCCTA AACTAGGAAC TGGCCTTAGT TTTGACAGCA CAGGTGCCAT
34001 TACAGTAGGA AACAAAAATA ATGATAAGCT AACTTTGTGG ACCACACCAG
34051 CTCCATCTCC TAACTGTAGA CTAAATGCAG AGAAAGATGC TAAACTCACT
34101 TTGGTCTTAA CAAAATGTGG CAGTCAAATA CTTGCTACAG TTTCAGTTTT
34151 GGCTGTTAAA GGCAGTTTGG CTCCAATATC TGGAACAGTT CAAAGTGCTC
34201 ATCTTATTAT AAGATTTGAC GAAAATGGAG TGCTACTAAA CAATTCCTTC
34251 CTGGACCCAG AATATTGGAA CTTTAGAAAT GGAGATCTTA CTGAAGGCAC
34301 AGCCTATACA AACGCTGTTG GATTTATGCC TAACCTATCA GCTTATCCAA
34351 AATCTCACGG TAAAACTGCC AAAAGTAACA TTGTCAGTCA AGTTTACTTA
34401 AACGGAGACA AAACTAAACC TGTAACACTA ACCATTACAC TAAACGGTAC
34451 ACAGGAAACA GGAGACACAA CTCCAAGTGC ATACTCTATG TCATTTTCAT
34501 GGGACTGGTC TGGCCACAAC TACATTAATG AAATATTTGC CACATCCTCT
34551 TACACTTTTT CATACATTGC CCAAGAATAA AGAATCGTTT GTGTTATGTT
34601 TCAACGTGTT TATTTTTCAA TTGCAGAAAA TTTCGAATCA TTTTTCATTC
34651 AGTAGTATAG CCCCACCACC ACATAGCTTA TACAGATCAC CGTACCTTAA
34701 TCAAACTCAC AGAACCCTAG TATTCAACCT GCCACCTCCC TCCCAACACA
34751 CAGAGTACAC AGTCCTTTCT CCCCGGCTGG CCTTAAAAAG CATCATATCA
34801 TGGGTAACAG ACATATTCTT AGGTGTTATA TTCCACACGG TTTCCTGTCG
34851 AGCCAAACGC TCATCAGTGA TATTAATAAA CTCCCCGGGC AGCTCACTTA
34901 AGTTCATGTC GCTGTCCAGC TGCTGAGCCA CAGGCTGCTG TCCAACTTGC
34951 GGTTGCTTAA CGGGCGGCGA AGGAGAAGTC CACGCCTACA TGGGGGTAGA
35001 GTCATAATCG TGCATCAGGA TAGGGCGGTG GTGCTGCAGC AGCGCGCGAA
35051 TAAACTGCTG CCGCCGCCGC TCCGTCCTGC AGGAATACAA CATGGCAGTG
35101 GTCTCCTCAG CGATGATTCG CACCGCCCGC AGCATAAGGC GCCTTGTCCT
35151 CCGGGCACAG CAGCGCACCC TGATCTCACT TAAATCAGCA CAGTAACTGC
35201 AGCACAGCAC CACAATATTG TTCAAAATCC CACAGTGCAA GGCGCTGTAT
35251 CCAAAGCTCA TGGCGGGGAC CACAGAACCC ACGTGGCCAT CATACCACAA
35301 GCGCAGGTAG ATTAAGTGGC GACCCCTCAT AAACACGCTG GACATAAACA
35351 TTACCTCTTT TGGCATGTTG TAATTCACCA CCTCCCGGTA CCATATAAAC
35401 CTCTGATTAA ACATGGCGCC ATCCACCACC ATCCTAAACC AGCTGGCCAA
35451 AACCTGCCCG CCGGCTATAC ACTGCAGGGA ACCGGGACTG GAACAATGAC
35501 AGTGGAGAGC CCAGGACTCG TAACCATGGA TCATCATGCT CGTCATGATA
35551 TCAATGTTGG CACAACACAG GCACACGTGC ATACACTTCC TCAGGATTAC
35601 AAGCTCCTCC CGCGTTAGAA CCATATCCCA GGGAACAACC CATTCCTGAA
35651 TCAGCGTAAA TCCCACACTG CAGGGAAGAC CTCGCACGTA ACTCACGTTG
35701 TGCATTGTCA AAGTGTTACA TTCGGGCAGC AGCGGATGAT CCTCCAGTAT
35751 GGTAGCGCGG GTTTCTGTCT CAAAAGGAGG TAGACGATCC CTACTGTACG
35801 GAGTGCGCCG AGACAACCGA GATCGTGTTG GTCGTAGTGT CATGCCAAAT
35851 GGAACGCCGG ACGTAGTCAT ATTTCCTGAA GCAAAACCAG GTGCGGGCGT
35901 GACAAACAGA TCTGCGTCTC CGGTCTCGCC GCTTAGATCG CTCTGTGTAG
35951 TAGTTGTAGT ATATCCACTC TCTCAAAGCA TCCAGGCGCC CCCTGGCTTC
36001 GGGTTCTATG TAAACTCCTT CATGCGCCGC TGCCCTGATA ACATCCACCA
36051 CCGCAGAATA AGCCACACCC AGCCAACCTA CACATTCGTT CTGCGAGTCA

36101 CACACGGGAG GAGCGGGAAG AGCTGGAAGA ACCATGTTTT TTTTTTTATT
36151 CCAAAAGATT ATCCAAAACC TCAAAATGAA GATCTATTAA GTGAACGCGC
36201 TCCCCTCCGG TGGCGTGGTC AAACTCTACA GCCAAAGAAC AGATAATGGC
36251 ATTTGTAAGA TGTTGCACAA TGGCTTCCAA AAGGCAAACG GCCCTCACGT
36301 CCAAGTGGAC GTAAAGGCTA AACCCTTCAG GGTGAATCTC CTCTATAAAC
36351 ATTCCAGCAC CTTCAACCAT GCCCAAATAA TTCTCATCTC GCCACCTTCT
36401 CAATATATCT CTAAGCAAAT CCCGAATATT AAGTCCGGCC ATTGTAAAAA
36451 TCTGCTCCAG AGCGCCCTCC ACCTTCAGCC TCAAGCAGCG AATCATGATT
36501 GCAAAAATTC AGGTTCCTCA CAGACCTGTA TAAGATTCAA AAGCGGAACA
36551 TTAACAAAAA TACCGCGATC CCGTAGGTCC CTTCGCAGGG CCAGCTGAAC
36601 ATAATCGTGC AGGTCTGCAC GGACCAGCGC GGCCACTTCC CCGCCAGGAA
36651 CCTTGACAAA AGAACCCACA CTGATTATGA CACGCATACT CGGAGCTATG
36701 CTAACCAGCG TAGCCCCGAT GTAAGCTTTG TTGCATGGGC GGCGATATAA
36751 AATGCAAGGT GCTGCTCAAA AAATCAGGCA AAGCCTCGCG CAAAAAAGAA
36801 AGCACATCGT AGTCATGCTC ATGCAGATAA AGGCAGGTAA GCTCCGGAAC
36851 CACCACAGAA AAAGACACCA TTTTTCTCTC AAACATGTCT GCGGGTTTCT
36901 GCATAAACAC AAAATAAAAT AACAAAAAAA CATTTAAACA TTAGAAGCCT
36951 GTCTTACAAC AGGAAAAACA ACCCTTATAA GCATAAGACG GACTACGGCC
37001 ATGCCGGCGT GACCGTAAAA AAACTGGTCA CCGTGATTAA AAAGCACCAC
37051 CGACAGCTCC TCGGTCATGT CCGGAGTCAT AATGTAAGAC TCGGTAAACA
37101 CATCAGGTTG ATTCACATCG GTCAGTGCTA AAAAGCGACC GAAATAGCCC
37151 GGGGGAATAC ATACCCGCAG GCGTAGAGAC AACATTACAG CCCCCATAGG
37201 AGGTATAACA AAATTAATAG GAGAGAAAAA CACATAAACA CCTGAAAAAC
37251 CCTCCTGCCT AGGCAAAATA GCACCCTCCC GCTCCAGAAC AACATACAGC
37301 GCTTCCACAG CGGCAGCCAT AACAGTCAGC CTTACCAGTA AAAAAGAAAA
37351 CCTATTAAAA AAACACCACT CGACACGGCA CCAGCTCAAT CAGTCACAGT
37401 GTAAAAAAGG GCCAAGTGCA GAGCGAGTAT ATATAGGACT AAAAAATGAC
37451 GTAACGGTTA AAGTCCACAA AAAACACCCA GAAAACCGCA CGCGAACCTA
37501 CGCCCAGAAA CGAAAGCCAA AAAACCCACA ACTTCCTCAA ATCGTCACTT
37551 CCGTTTTCCC ACGTTACGTC ACTTCCCATT TTAAGAAAAC TACAATTCCC
37601 AACACATACA AGTTACTCCG CCCTAAAACC TACGTCACCC GCCCCGTTCC
37651 CACGCCCCGC GCCACGTCAC AAACTCCACC CCCTCATTAT CATATTGGCT
37701 TCAATCCAAA ATAAGGTATA TTATTGATGA TGTTAATTAA TTTAAATCCG
37751 CATGCGATAT CGAGCTCTCC CGGGAATTCG GATCTGCGAC GCGAGGCTGG
37801 ATGGCCTTCC CCATTATGAT TCTTCTCGCT TCCGGCGGCA TCGGGATGCC
37851 CGCGTTGCAG GCCATGCTGT CCAGGCAGGT AGATGACGAC CATCAGGGAC
37901 AGCTTCACGG CCAGCAAAAG GCCAGGAACC GTAAAAAGGC CGCGTTGCTG
37951 GCGTTTTTCC ATAGGCTCCG CCCCCCTGAC GAGCATCACA AAAATCGACG
38001 CTCAAGTCAG AGGTGGCGAA ACCCGACAGG ACTATAAAGA TACCAGGCGT
38051 TTCCCCCTGG AAGCTCCCTC GTGCGCTCTC CTGTTCCGAC CCTGCCGCTT
38101 ACCGGATACC TGTCCGCCTT TCTCCCTTCG GGAAGCGTGG CGCTTTCTCA
38151 ATGCTCACGC TGTAGGTATC TCAGTTCGGT GTAGGTCGTT CGCTCCAAGC
38201 TGGGCTGTGT GCACGAACCC CCCGTTCAGC CCGACCGCTG CGCCTTATCC
38251 GGTAACTATC GTCTTGAGTC CAACCCGGTA AGACACGACT TATCGCCACT
38301 GGCAGCAGCC ACTGGTAACA GGATTAGCAG AGCGAGGTAT GTAGGCGGTG
38351 CTACAGAGTT CTTGAAGTGG TGGCCTAACT ACGGCTACAC TAGAAGGACA
38401 GTATTTGGTA TCTGCGCTCT GCTGAAGCCA GTTACCTTCG GAAAAAGAGT
38451 TGGTAGCTCT TGATCCGGCA AACAAACCAC CGCTGGTAGC GGTGGTTTTT
38501 TTGTTTGCAA GCAGCAGATT ACGCGCAGAA AAAAAGGATC TCAAGAAGAT
38551 CCTTTGATCT TTTCTACGGG GTCTGACGCT CAGTGGAACG AAAACTCACG
38601 TTAAGGGATT TTGGTCATGA GATTATCAAA AAGGATCTTC ACCTAGATCC
38651 TTTTAAATCA ATCTAAAGTA TATATGAGTA AACTTGGTCT GACAGTTACC
38701 AATGCTTAAT CAGTGAGGCA CCTATCTCAG CGATCTGTCT ATTTCGTTCA
38751 TCCATAGTTG CCTGACTCCC CGTCGTGTAG ATAACTACGA TACGGGAGGG
38801 CTTACCATCT GGCCCCAGTG CTGCAATGAT ACCGCGAGAC CCACGCTCAC
38851 CGGCTCCAGA TTTATCAGCA ATAAACCAGC CAGCCGGAAG GGCCGAGCGC
38901 AGAAGTGGTC CTGCAACTTT ATCCGCCTCC ATCCAGTCTA TTAATTGTTG
38951 CCGGGAAGCT AGAGTAAGTA GTTCGCCAGT TAATAGTTTG CGCAACGTTG
39001 TTGCCATTGN TGCAGGCATC GTGGTGTCAC GCTCGTCGTT TGGTATGGCT
39051 TCATTCAGCT CCGGTTCCCA ACGATCAAGG CGAGTTACAT GATCCCCCAT
39101 GTTGTGCAAA AAAGCGGTTA GCTCCTTCGG TCCTCCGATC GTTGTCAGAA
39151 GTAAGTTGGC CGCAGTGTTA TCACTCATGG TTATGGCAGC ACTGCATAAT
39201 TCTCTTACTG TCATGCCATC CGTAAGATGC TTTTCTGTGA CTGGTGAGTA

39251  CTCAACCAAG TCATTCTGAG AATAGTGTAT GCGGCGACCG AGTTGCTCTT
39301  GCCCGGCGTC AACACGGGAT AATACCGCGC CACATAGCAG AACTTTAAAA
39351  GTGCTCATCA TTGGAAAACG TTCTTCGGGG CGAAAACTCT CAAGGATCTT
39401  ACCGCTGTTG AGATCCAGTT CGATGTAACC CACTCGTGCA CCCAACTGAT
39451  CTTCAGCATC TTTTACTTTC ACCAGCGTTT CTGGGTGAGC AAAAACAGGA
39501  AGGCAAAATG CCGCAAAAAA GGGAATAAGG GCGACACGGA AATGTTGAAT
39551  ACTCATACTC TTCCTTTTTC AATATTATTG AAGCATTTAT CAGGGTTATT
39601  GTCTCATGAG CGGATACATA TTTGAATGTA TTTAGAAAAA TAAACAAATA
39651  GGGGTTCCGC GCACATTTCC CCGAAAAGTG CCACCTGACG TCTAAGAAAC
39701  CATTATTATC ATGACATTAA CCTATAAAAA TAGGCGTATC ACGAGGCCCT
39751  TTCGTCTTCA AGGATCCGAA TTCCCGGGAG AGCTCGATAT CGCATGCGGA
39801  TTTAAATTAA TTAA
//

## Claims

1. A replication defective adenoviral vector comprising
(i) a nucleic acid encoding at least one SARS-CoV-2 spike protein or a derivative thereof and
(ii) a nucleic acid encoding at least one SARS-CoV-2 nucleocapsid protein or a derivative thereof,
wherein the adenoviral vector displays a polypeptide comprising a receptor-binding domain (RBD) of a SARS-CoV-2 spike protein or a derivative thereof via protein IX (pIX) on its capsid.

2. The adenoviral vector according to claim 1, wherein the at least one SARS-CoV-2 spike protein of (i) is selected from spike proteins of any known SARS-CoV viruses derived from the Wuhan variant and derivatives thereof, in particular spike proteins of SARS-CoV-2 variants of concern, such as Alpha, Beta, Gamma, Delta and Omicron variants, more particularly wherein the at least one SARS-CoV-2 spike protein of (i) comprises the spike protein of SARS-CoV-2 omicron variant or a derivative thereof, SARS-CoV-2 Delta plus variant or a derivative thereof, or combinations thereof.

3. The adenoviral vector according to claim 2, wherein the at least one SARS-CoV-2 spike protein of (i) is encoded by a nucleic acid sequence comprising nucleotides 1131 to 4931 as shown in SEQ IN NO:1.

4. The adenoviral vector according to any one of claims 1-3, wherein the at least one nucleocapsid protein of (ii) is selected from nucleocapsid proteins of any known SARS-CoV viruses derived from the Wuhan variant and derivatives thereof, in particular nucleocapsid proteins of SARS-CoV-2 variants of concern, such as Alpha, Beta, Gamma, Delta and Omicron variants, more particularly wherein the at least one nucleocapsid protein of (ii) comprises the nucleocapsid protein of SARS-CoV-2 Omicron variant or a derivative thereof, SARS-CoV-2 delta plus variant or a derivative thereof, or combinations thereof.

5. The adenoviral vector according to claim 4, wherein the nucleocapsid protein of (iii) is encoded by a nucleic acid sequence comprising nucleotides 4995 to 6242 of SEQ ID NO:1.

6. The adenoviral vector according to any one of claims 1-5, wherein the RBD displayed on the capsid is selected from RBDs of any known SARS-CoV viruses derived from the Wuhan variant and derivatives thereof, in particular RBDs of SARS-CoV-2 variants of concern, such as Alpha, Beta, Gamma, Delta and Omicron variants, more particularly wherein the RBD displayed on the capsid is the RBD of SARS-CoV-2 delta plus variant or a derivative thereof.

7. The adenoviral vector according to claim 6, wherein the RBD displayed on the capsid comprises a protein encoded by a nucleic acid sequence comprising nucleotides 7031 to 7699 of SEQ ID NO:1.

8. The adenoviral vector according to any one of claims 1-7, wherein the adenovirus is derived from human or chimpanzee serotype 5, 26, 35 or 3, in particular human Adenovirus 5 serotype.

9. The adenoviral vector according to any one of claims 1-8, comprising deletions of the E1 and E3 regions.

10. The adenoviral vector according to any one of claims 1-9 comprising a further a nucleic acid encoding at least one protein or derivative thereof of a microorganism, which is different from SARS-CoV-2 and which is preferably responsible for an infectious disease.

11. The adenoviral vector according to any one of claims 1-10, for use in prevention or treating a COVID-19 disease.

12. The adenoviral vector for use according to claim 11, wherein the vector is administered by injection, in particular by intramuscular or subcutaneous injection, and/or wherein the vector is administered to the respiratory tract of a subject, preferably to the mucosa of the respiratory tract, in particular via intranasal or oral administration.

13. A vaccine composition comprising the adenoviral vector according to any one of claims 1-12, and optionally one or more further active agents, i.e. in particular further immunogenicity-conferring agents.

14. The vaccine composition according to claim 13, adapted
(i) for administration by injection, in particular adapted for intramuscular or subcutaneous injection, and/or
(ii) for administration to the respiratory tract of a subject, preferably to the mucosa of the respiratory tract, in particular for intranasal or oral administration.

15. A method of boosting an antigen-specific immune response in a subject comprising administering to said subject a sufficient amount of the adenoviral vector according to any one of claims 1-10 or the vaccine composition according to claim 13 or 14.

16. The method according to claim 15, wherein the vector or vaccine composition is administered by injection, in particular by intramuscular or subcutaneous injection, and/or wherein the vector or vaccine composition is administered to the respiratory tract of a subject, preferably to the mucosa of the respiratory tract, in particular via intranasal or oral administration.

17. Platform for providing multivalent vaccines
comprising an adenoviral vector according to any one of claims 1-10, in particular claim 10.
